# EUROPÄISCHE PATENTANMELDUNG

(11) **EP 3 905 196 A1**
(43) Veröffentlichungstag der Anmeldung: **03.11.2021**
(21) Anmeldenummer: 21162775.7
(22) Anmeldetag: 16.03.2021
(51) Int. Cl.: G06T 7/62, G06T 7/73, A43D 1/02, A61B 5/107, G06Q 30/06, G01C 11/06

(54) **ERFASSEN ANATOMISCHER MASSE UND BESTIMMEN PASSENDER KLEIDUNGSSTÜCKE**

(30) Priorität: 30.04.2020 DE 102020205563
(71) Anmelder: Footprint Technologies GmbH, 10245 Berlin (DE)
(72) Erfinder: BRENDEL, Matthias, 10245 Berlin (DE); KLEINERT, Carolin, 10435 Berlin (DE); KEZZE, Muhammad Ali, 40589 Düsseldorf (DE); PRADA GONZÁLES, Andrés, 10557 Berlin (DE)
(74) Vertreter: Maiwald Patent- und Rechtsanwaltsgesellschaft mbH

(57) **Zusammenfassung**

Die Erfindung betrifft ein computer-implementiertes Verfahren zur Bestimmung eines anatomischen Maßes. Dazu werden ein oder mehrere Bilder verwendet, die zumindest einen Körperteil und ein Referenzobjekt mit bekannten Abmessungen zeigen. Die Bilder werden mittels eines konvolutionalen neuronalen Netzes segmentiert, um den Körperteil, das Referenzobjekt sowie sonstige Bildabschnitte zu unterscheiden. Nachfolgend können Eckpunkte einer Grundfläche des Referenzobjekts in den segmentierten Bildern bestimmt, Ausreißer unter den ermittelten Eckpunkten verworfen und entzerrte Bilder auf der Grundlage von ermittelten Eckpunkten bestimmt werden. Ferner können Messwerte für das anatomische Maß auf der Grundlage der entzerrten Bilder bestimmt werden, und das Endergebnis für das anatomische Maß kann in robuster Weise auf der Grundlage der Messwerte bestimmt werden.

## Beschreibung

### GEBIET DER ERFINDUNG

Die Erfindung betrifft ein computer-implementiertes Verfahren zur Bestimmung eines anatomischen Maßes, bei dem ein konvolutionales neuronales Netz verwendet wird, um in einem digitalen Bild einen Körperteil eines Menschen, ein Referenzobjekt mit bekannten Abmessungen sowie sonstige Bildabschnitte voneinander zu unterscheiden. Darüber hinaus betrifft die Erfindung ein Verfahren zur Bestimmung eines Schuhmodells und einer Schuhgröße, umfassend das Messen einer Schuhinnenlänge, einer Schuhinnenbreite und/oder einer Schuhinnenhöhe für eine Vielzahl von Schuhmodellen und Schuhgrößen.

### STAND DER TECHNIK

Die höchsten Retourenquoten im Onlinehandel treten bei Kleidungsstücken auf, weil meist mehrere Größen bestellt und nur die tatsächlich passende Größe behalten wird. Zur Vermeidung dieser Retouren müssen im Wesentlichen zwei Probleme gelöst werden. Zunächst müssen ein oder mehrere Maße des Körpers oder eines Körperteils bestimmt werden. Danach muss auf der Grundlage der Messergebnisse ein passendes Kleidungsstück bestimmt werden.

Als Kleidung wird hier die Gesamtheit aller Materialien bezeichnet, die als künstliche Hülle den Körper des Menschen mehr oder weniger enganliegend umgeben. Insbesondere gehören zu Kleidung auch Schuhe und Kopfbedeckungen wie zum Beispiel Helme.

Für das erste Problem, d. h. für das Bestimmen anatomischer Maße wurden verschiedene Vorrichtungen entwickelt. Beispielsweise wird in der US 2006/0140463 A1 eine Vorrichtung zum Vermessen von Körpern oder Körperteilen beschrieben, wobei der Körper oder der Körperteil mit einem elastischen Überzug bekleidet ist, der kontrastreiche, fotogrammmetrisch auswertbare Marken aufweist und aus verschiedenen Aufnahmepositionen fotografiert wird. Hierzu wird mindestens eine Kamera um den Körper bzw. den Körperteil herumgeführt, um sich überlappende Aufnahmen aus verschiedenen Raumpositionen zu erstellen, welche sowohl den Körper bzw. den Körperteil als auch mehrere Marken auf einer Unterlage zeigen.

Speziell für die Vermessung von Körpern oder Körperteilen entwickelte Vorrichtungen sind jedoch mit erheblichen Kosten verbunden und daher für den Hausgebrauch kaum geeignet. Verschiedene Verfahren wurden vorgeschlagen, um das Vermessen von Körpern oder Körperteilen auch ohne aufwändige dedizierte Vorrichtungen zu ermöglichen. Beispielsweise kann die Länge eines Fußes bestimmt werden, indem der Umriss des Fußes auf ein Blatt Papier gezeichnet und die Länge des Fußes anhand der Umrisslinie gemessen wird. Derlei Verfahren können jedoch mit signifikanten Messfehlern behaftet sein. Beispielsweise können Messfehler daraus resultieren, dass der Stift beim Zeichnen der Umrisslinie des Fußes nicht senkrecht zu der Papieroberfläche gehalten wird.

Darüber hinaus wurden Verfahren unter Verwendung einer Smartphone-Kamera vorgeschlagen, um die Vermessung von Körpern oder Körperteilen zu vereinfachen. Beispielsweise wird in der EP 2641539 A1 ein Verfahren zum Bestimmen der Abmessungen eines Körperteils beschrieben, bei dem der Körperteil oder der auf einer Darstellungsfläche aufgezeichnete Umriss des Körperteils und ein Referenzobjekt mit bekannten Abmessungen fotografiert werden, wobei der Körperteil oder dessen Umriss und das Referenzobjekt auf demselben Foto abgebildet sind. Die Abmessungen des Körperteils werden dann anhand der Fotografie basierend auf den bekannten Abmessungen des Referenzobjekts errechnet.

Nach dem Bestimmen der Maße des Körpers oder des Körperteils wird in der Regel eine Größentabelle verwendet, um die passende Kleidungsgröße zu ermitteln. Beispielsweise werden Längentabellen für Schuhe verwendet, um auf der Grundlage der gemessenen Länge des Fußes eine passende Schuhgröße zu ermitteln.

### OFFENBARUNG DER ERFINDUNG

Bei den bekannten Verfahren zum Bestimmen anatomischer Maße unter Verwendung einer Smartphone-Kamera treten mitunter erhebliche Messfehler auf. Die Messfehler können aufgrund einer ungleichmäßigen Ausleuchtung des Körpers oder Körperteils während der Bildaufnahme auftreten. Insbesondere kann die ungleichmäßige Ausleuchtung zu Schattenwürfen führen, die wiederum zu Fehlern in den Ergebnissen der Bildverarbeitungsverfahren führen. Fehler bei der Bestimmung anatomischer Maße können auch dadurch verursacht werden, dass signifikante Bereiche des Körperteils oder des Referenzobjekts in dem Bild verdeckt sind, beispielsweise durch das Bein bei der Vermessung eines Fußes. Es ist daher wünschenswert, ein robustes Verfahren zur Bestimmung anatomischer Maße auf der Grundlage eines oder mehrerer von einer Kamera aufgenommener Bilder bereitzustellen.

Bei der Bestimmung eines passenden Kleidungsstücks auf der Grundlage eines oder mehrerer gemessener anatomischer Maße entstehen Fehler dadurch, dass die Größen von Kleidungsstücken und insbesondere Schuhgrößen nicht genormt sind, sodass die Schuhgröße nicht zur eindeutigen Bestimmung der Schuhinnenlänge verwendet werden kann. Das Finden von Kleidungsstücken, die zur Anatomie eines Kunden passen, ist insbesondere bei Schuhen sehr mühsam und mit hohem Zeitaufwand verbunden. Die Schuhgrößen werden in der Regel nur mit einer Längenangabe und selten, zum Beispiel bei Kinderschuhen, auch einer Breitenangabe beschrieben. Da es zwischen Herstellern und Modellen zu erheblichen Größenschwankungen kommt, ist die Wahrscheinlichkeit, mit den bekannten Verfahren einen passenden Schuh zu bestimmen, begrenzt. Es ist daher wünschenswert, die Zuordnung anatomischer Maße zu Modellen und Größen von Kleidungsstücken und insbesondere Schuhen zu verbessern.

Eine übergeordnete Aufgabe besteht darin, die mit hohen Kosten und einer erheblichen Umweltbelastung einhergehenden Retouren im Onlinehandel von Kleidungsstücken zu reduzieren.

Ein erster Aspekt der Erfindung betrifft ein computer-implementiertes Verfahren zur Bestimmung eines anatomischen Maßes. Dazu wird ein erstes Bild geladen, das zumindest einen Körperteil und ein Referenzobjekt mit bekannten Abmessungen zeigt, wobei eine Distanz des Referenzobjekts zu dem Körperteil in einer Bildaufnahmerichtung des ersten Bildes bekannt ist. Ein erstes segmentiertes Bild wird bestimmt, indem ein konvolutionales neuronales Netz auf das erste Bild angewendet wird, wobei das konvolutionale neuronale Netz dazu ausgeführt ist, den Körperteil, das Referenzobjekt sowie sonstige Bildabschnitte zu unterscheiden. Das anatomische Maß wird dann auf der Grundlage des ersten segmentierten Bildes, der bekannten Abmessungen des Referenzobjekts und der bekannten Distanz des Referenzobjekts zu dem Körperteil in der Bildaufnahmerichtung bestimmt.

Das erste Bild ist insbesondere ein digitales Bild, das von einer Kamera aufgenommen wurde, zum Beispiel der Kamera eines Smartphones. Das erfindungsgemäße Verfahren kann beispielsweise von einem Applikationsprozessor des Smartphones ausgeführt werden. Es ist aber auch möglich, dass das erste Bild beispielsweise von einem Smartphone zu einer Datenverarbeitungsvorrichtung übertragen wird und die Datenverarbeitungsvorrichtung das erfindungsgemäße Verfahren ausführt. Bei der Datenverarbeitungsvorrichtung kann es sich um einen lokalen oder fernen (remote) Server handeln.

Das voranstehend und nachfolgend beschriebene Verfahren kann insbesondere browserbasiert durchgeführt werden. Mit einem Bediengerät, beispielsweise einem Smartphone einem PC, einem Laptop, einem Tablet-PC, einem Smartdevice oder dergleichen, kann dabei eine browserbasierte Applikation, welche vom Server bereitgestellt werden kann, gestartet werden, um das erfindungsgemäße Verfahren durchzuführen. Die Applikation kann beispielsweise JAVA-Script und/oder Type-Script basiert sein.

Durch das Laden des ersten Bildes wird das Bild beispielsweise von einer Speichereinheit oder einer Kommunikationseinheit eines Datenverarbeitungssystems bereitgestellt. Bei der Speichereinheit kann es sich um eine flüchtige oder eine nichtflüchtige Speichereinheit handeln.

Das erste Bild enthält eine Abbildung von zumindest einem Körperteil eines Menschen. Möglicherweise enthält das erste Bild eine Abbildung des gesamten Körpers eines Menschen.

Ferner enthält das erste Bild eine Abbildung eines Referenzobjekts mit bekannten Abmessungen. Bei dem Referenzobjekt kann es sich beispielsweise um einen Gegenstand mit genormter Größe wie zum Beispiel ein DIN-A4-formatiges Blatt Papier handeln. Die Distanz des Referenzobjekts zu dem Körperteil in einer Blickrichtung der Kamera während der Aufnahme des ersten Bildes ist bei der Ausführung des computerimplementierten Verfahrens bekannt. Insbesondere ist bekannt, ob und wie weit sich der Körperteil von der das erste Bild aufnehmenden Kamera aus gesehen vor oder hinter dem Referenzobjekt befindet. Beispielsweise kann es sich bei dem Körperteil um einen Fuß handeln, der auf einem Blatt DIN-A4-Blatt Papier als Referenzobjekt steht. In diesem Fall wäre die Distanz des Referenzobjekts zu dem Fuß gleich Null.

Bei dem ersten Bild kann es sich insbesondere um ein Farb- oder ein Graustufenbild handeln. Beispielsweise kann es sich bei dem ersten Bild um ein Farbbild unter Verwendung des RGB-Farbraums handeln. Die Erfindung ist jedoch nicht auf Farbbilder, Farbräume oder Farbmodelle beschränkt.

Das konvolutionale neuronale Netz ist dazu ausgeführt, eine semantische Segmentierung des ersten Bildes zu bestimmen. Ein Pixel des ersten segmentierten Bildes indiziert, ob ein entsprechendes Pixel des ersten Bildes den Körperteil, das Referenzobjekt oder einen sonstigen Gegenstand abbildet. Das konvolutionale neuronale Netz kann also dazu ausgeführt sein, wenigstens drei Objektklassen zu unterscheiden. Dementsprechend können die Pixelwerte des ersten segmentierten Bildes 0, 1 oder 2 sein. Das konvolutionale neuronale Netz kann auch zur Erkennung weiterer Objektklassen ausgeführt sein. Beispielsweise kann das konvolutionale neuronale Netz dazu ausgeführt sein, in dem ersten segmentierten Bild einen Fuß, ein Bein, das Referenzobjekt sowie sonstige Gegenstände zu unterscheiden.

Das konvolutionale neuronale Netz kann mehrere konvolutionale Schichten und/oder mehrere Pooling-Schichten aufweisen. Das Training des konvolutionalen neuronalen Netzes erfolgt vorzugsweise überwacht. Dies kann beispielsweise dadurch erfolgen, dass Bilder händisch segmentiert werden, um Trainingsdaten zu generieren. Um den Aufwand für das Training des konvolutionalen neuronalen Netzes zu reduzieren, können Bilder unter Verwendung spezieller Algorithmen vorsegmentiert werden. Die vorsegmentierten Bilder können geprüft und gegebenenfalls manuell korrigiert werden, bevor sie für das Training des konvolutionalen Netzes verwendet werden.

Das Verfahren kann einen Schritt des Segmentierens des ersten Bildes und/oder weiterer Bilder umfassen. Das Segmentieren kann dabei ein Zuordnen von Pixeln des ersten Bildes und/oder weiterer Bilder zu dem Körperteil, dem Referenzobjekt und/oder einem sonstigen Gegenstand umfassen. Alternativ oder zusätzlich kann das Segmentieren ein Bestimmen, Ermitteln und/oder Identifizieren eines dem Körperteil, dem Referenzobjekt und/oder dem weiteren Gegenstand zuzuordnenden Bildbereichs (und/oder zuzuordnender Pixel) des ersten Bildes und/oder weiterer Bilder umfassen.

Das anatomische Maß wird dann auf der Grundlage des ersten segmentierten Bildes, der bekannten Abmessungen des Referenzobjekts und der bekannten Distanz des Referenzobjekts zu dem Körperteil in der Bildaufnahmerichtung bestimmt.

Da das konvolutionale neuronale Netz von bekannten dreidimensionalen Modellen von Körperteilen keinen Gebrauch macht, ist die Bestimmung des anatomischen Maßes durch das erfindungsgemäße Verfahren in manchen Fällen weniger präzise als dies bei Verwendung bekannter dreidimensionaler Modelle von Körperteilen möglich wäre. Bei der Bestimmung anatomischer Maße in privaten Räumen kommt es jedoch nicht selten vor, dass der Körperteil bei der Aufnahme des Bildes schlecht ausgeleuchtet ist, insbesondere dass der Körperteil ungleichmäßig ausgeleuchtet ist, was zu Schattenwürfen führen kann. Ferner können zum Beispiel bei der Aufnahme eines Bildes von einem Fuß Abschnitte des Bildes durch das Bein verdeckt sein. Aufgrund der Verwendung eines konvolutionalen neuronalen Netzes bietet das erfindungsgemäße Verfahren im Vergleich mit bekannten Verfahren zur Bestimmung anatomischer Maße den Vorteil, dass die Messwerte robust sind im Hinblick auf eine schlechte Ausleuchtung des Körperteils sowie im Hinblick auf verdeckte Bildabschnitte. Durch die Verwendung des konvolutionalen neuronalen Netzes ist das erfindungsgemäße Verfahren geeignet für die zuverlässige Bestimmung anatomischer Maße selbst wenn der zu vermessende Körperteil bei der Aufnahme des ersten Bildes schlecht ausgeleuchtet oder teilweise verdeckt ist.

Gemäß einer Ausführungsform weist das Referenzobjekt eine polygonale Grundfläche mit bekannten Abmessungen auf. Ferner umfasst das Verfahren das Bestimmen von ersten Eckpunkten der Grundfläche des Referenzobjekts in dem ersten segmentierten Bild. Weiterhin wird durch Entzerren des ersten segmentierten Bildes unter Verwendung der ersten Eckpunkte ein erstes entzerrtes Bild bestimmt. Das anatomische Maß wird dann auf der Grundlage des ersten entzerrten Bildes, der bekannten Abmessungen des Referenzobjekts und der bekannten Distanz des Referenzobjekts zu dem Körperteil in der Bildaufnahmerichtung bestimmt.

Die Grundfläche des Referenzobjekts kann beispielsweise ein Parallelogramm oder ein Rechteck sein. Ferner kann die Grundfläche des Referenzobjekts eine genormte Größe wie zum Beispiel ein DIN-A4-Format aufweisen. Andere polygonale Formen der Grundfläche sind ebenfalls möglich. Darüber hinaus ist es möglich, dass eine polygonale Form auf einem größeren Gegenstand wie zum Beispiel einem Fußboden markiert ist. Beispielsweise könnte auf einem Fußboden ein DIN-A4-formatiges ausgefülltes Rechteck markiert sein. In diesem Fall kann die DIN-A4-formatige Farbschicht als das Referenzobjekt angesehen werden.

Eine Vielzahl bekannter Algorithmen zur Erkennung von Eckpunkten kann verwendet werden, um die Eckpunkte der Grundfläche in dem ersten segmentierten Bild zu bestimmen. Ein Beispiel ist der von Harris und Stephens entwickelte Harris-Eckpunkt-Detektor. Für den Fall, dass die Grundfläche des Referenzobjekts durch zwei Parallelenpaare begrenzt wird, wird unten ein weiterer, besonders vorteilhafter Algorithmus beschrieben.

Auf der Grundlage von mindestens drei Eckpunkten der polygonalen Grundfläche in dem ersten segmentierten Bild und den bekannten Abmessungen der Grundfläche kann eine perspektivische Entzerrung des ersten segmentierten Bildes berechnet werden. Vorzugsweise wird die perspektivische Entzerrung des ersten segmentierten Bildes auf der Grundlage von vier Eckpunkten der Grundfläche in dem ersten segmentierten Bild und den bekannten Abmessungen der Grundfläche bestimmt. Das perspektivische Entzerren kann beispielsweise dazu ausgeführt sein, dass parallele Kanten der Grundfläche des Referenzobjekts in dem ersten entzerrten Bild parallel verlaufen.

Wenn das Referenzobjekt eine rechteckige Grundfläche aufweist, kann nach dem der perspektivischen Entzerrung zusätzlich ein Beschränken des Bildausschnitts auf die rechteckige Grundfläche erfolgen.

Wenn beispielsweise das erste Bild einen Fuß wiedergibt, der auf einem DIN-A4-formatigen Blatt Papier als Referenzobjekt steht, so kann unter Verwendung des entzerrten Bildes und der bekannten Abmessungen des Referenzobjekts eine Länge, eine Breite und/oder eine Höhe des Fußes bestimmt werden.

Wenn das A4-Blatt nicht perfekt planar auf dem Untergrund liegt, weil es beispielsweise Faltmarken aufweist oder sich aufgrund von Fußschweiß des zu vermessenden Probanden wellt, kann dies vom Server (z.B. algorithmisch) erkannt werden und die Referenzabmessung des Blattes kann um einen der Abweichung zu einem planar aufliegenden DIN-A4-Blatt entsprechenden und/oder zur Abweichung proportionalen Korrekturfaktor korrigiert werden.

Gemäß einer Ausführungsform wird die Grundfläche des Referenzobjekts durch zwei Parallelenpaare begrenzt. Somit kann die Grundfläche des Referenzobjekts die Form eines Parallelogramms und vorzugsweise die Form eines Rechtecks aufweisen. Ferner umfasst das Bestimmen der ersten Eckpunkte der Grundfläche in dem ersten segmentierten Bild das Bestimmen von vier Kanten der Grundfläche in dem ersten segmentierten Bild, das Zuordnen jeder der vier Kanten zu einem ersten oder einem zweiten Parallelenpaar und das Bestimmen von ersten Schnittpunkten zwischen jeweils einer Kante des ersten Parallelenpaars und einer Kante des zweiten Parallelenpaars.

Somit kann die Grundfläche des Referenzobjekts die Form eines Parallelogramms und vorzugsweise die Form eines Rechtecks aufweisen.

In dem ersten Bild können Eckpunkte der Grundfläche des Referenzobjekts verdeckt sein. Beispielsweise kann bei der Aufnahme eines Fußes eine Ecke der Grundfläche durch ein Bein verdeckt sein. Wenn jedoch der Verlauf der vier Kanten der Grundfläche in dem ersten segmentierten Bild zumindest teilweise abgebildet ist, können die Ecken der Grundfläche als Schnittpunkte der vier Kanten bestimmt werden. Dazu können für eine Kante ein auf der Kante liegender Punkt sowie eine Steigung bestimmt werden. Die vier Kanten der Grundfläche des Referenzobjekts in dem ersten segmentierten Bild können mittels bekannter Kantendetektionsverfahren bestimmt werden.

Die vier Kanten der Grundfläche des Referenzobjekts bilden zwei Parallelenpaare. Nachdem der Verlauf der vier Kanten bestimmt wurde, kann jede der vier Kanten einem von zwei Parallelenpaaren zugeordnet werden. Dies kann das Auswählen einer ersten Kante und das Bestimmen einer zweiten Kante umfassen, wobei die zweite Kante zu der ersten Kante den geringsten Winkel oder die ähnlichste Steigung aufweist, und somit zu der ersten Kante am ehesten parallel verläuft.

Die ersten Schnittpunkte zwischen jeweils einer Kante des ersten Parallelenpaars und einer Kante des zweiten Parallelenpaars können als die zu bestimmenden ersten Eckpunkte der Grundfläche in dem ersten segmentierten Bild verwendet werden. Es ist aber auch möglich, dass die ersten Schnittpunkte verarbeitet werden und die verarbeiteten ersten Schnittpunkte als die zu bestimmenden ersten Eckpunkte der Grundfläche in dem ersten segmentierten Bild verwendet werden.

Gemäß einer weiteren Ausführungsform umfasst das Verfahren ferner das Detektieren von Fehlern bei der Bestimmung des ersten segmentierten Bildes. Wenn beispielsweise bekannt ist, dass die Grundfläche des Referenzobjekts die Form eines Rechtecks aufweist, der zu vermessende Körperteil ein Fuß ist und der Fuß bei der Aufnahme des ersten Bildes mittig auf der Grundfläche des Referenzobjekts steht, so unterbrechen Körperteile (Fuß oder Bein) den rechteckförmigen Umriss der Grundfläche des Referenzobjekts in dem ersten Bild in höchstens einem Bereich. Beispielsweise aufgrund eines Schattenwurfs kann es jedoch sein, dass der rechteckförmige Umriss der Grundfläche in dem mit dem konvolutionalen neuronalen Netz bestimmten ersten segmentierten Bild in mehreren Bereichen von als Körperteil klassifizierten Bildabschnitten unterbrochen ist. Dies kann als Fehler bei der Bestimmung des ersten segmentierten Bildes detektiert werden.

Bei der Detektion eines solchen Fehlers kann das segmentierte Bild verworfen werden, d.h. für dieses Bild wird beispielsweise keine Entzerrung ausgeführt. Weiterhin können zusätzliche Bilder geladen werden, wenn ein Fehler bei der Bestimmung des ersten segmentierten Bildes detektiert wird, insbesondere um ein verworfenes segmentiertes Bild zu ersetzen.

Gemäß einer weiteren Ausführungsform umfasst das Verfahren ferner das Laden weiterer Bilder, wobei die weiteren Bilder den Körperteil und das Referenzobjekt mit der bekannten Distanz in der Bildaufnahmerichtung zeigen. Durch separates Anwenden des konvolutionalen neuronalen Netzes auf die weiteren Bilder werden weitere segmentierte Bilder bestimmt. Ferner werden weitere Eckpunkte der Grundfläche des Referenzobjekts in den weiteren segmentierten Bildern bestimmt. Außerdem werden weitere entzerrte Bilder durch separates Entzerren von zumindest einem Teil der weiteren segmentierten Bilder unter Verwendung der weiteren Eckpunkte bestimmt. Weiterhin werden Messwerte des anatomischen Maßes separat für das erste entzerrte Bild und die weiteren entzerrten Bilder bestimmt, und zwar auf der Grundlage der bekannten Abmessungen des Referenzobjekts und der bekannten Distanz des Referenzobjekts zu dem Körperteil in der Bildaufnahmerichtung. Das anatomische Maß wird dann auf der Grundlage der Messwerte bestimmt.

Das erste Bild und/oder die weiteren Bilder können beispielsweise Einzelbilder eines Videos sein. Das Video kann von einem Smartphone aufgenommen worden sein. Um beispielsweise eine Länge, eine Breite und eine Höhe eines Fußes zu bestimmen, kann eine Kamera auf einem Viertelkreisbogen und/oder in einem gewissen Winkelbereich (beispielsweise rund 30° bis rund 180°, insbesondere rund 90° bis 135°) um den zu vermessenden Fuß herumgeschwenkt werden. Dabei wird die Kamera vorzugsweise in der Frontalebene der Person geführt, deren Fuß zu vermessen ist. Der Viertelkreisbogen kann einen kranial zu dem abzubildenden Fuß liegenden Punkt mit einem lateral zu dem abzubildenden Fuß liegenden Punkt verbinden. Der kranial zu dem abzubildenden Fuß liegende Punkt befindet sich vorzugsweise in der Nähe des Knies der Person, deren Fuß zu vermessen ist. Während der Aufnahme des Videos bleibt die Kamera-Blickrichtung vorzugsweise auf den Fuß gerichtet.

Die Führung entlang eines Viertelkreisbogens und/oder den Winkelbereich um den Fuß herum muss nicht genau eingehalten werden und dient allenfalls als grobe Richtlinie. Die Aufnahme des Videos kann vereinfacht werden, indem die Kamera von einer anderen Person um den zu vermessenden Fuß herumgeschwenkt wird.

Die Kamera kann dabei aus Sicht der Person links oder rechts (bzw. innen oder außen) um den Fuß herumgeführt werden. Alternativ oder zusätzlich kann die Kamera ausgehend vom kranialen Punkt und/oder ausgehend von einer Draufsicht auf das Körperteil, beispielsweise einer Draufsicht auf den Fußballen und/oder den Vorderfuß, innen oder außen um das Körperteil und/oder den Fuß herumgeführt werden. Optional kann die Kamera dabei bis zu einer Position, in welcher eine Ferse des Fußes erkennbar ist, und/oder zu einer Position, in der mehrere Ecken des Referenzobjekts, beispielsweise wenigstens drei Ecken oder vier Ecken des Referenzobjekts, in dem jeweiligen Bild erkennbar sind.

Die Videosequenz kann 20, 50, 100, 200 oder mehr Einzelbilder umfassen. Die Aufnahmepositionen der Einzelbilder können sich voneinander unterscheiden, wenn die Kamera während der Aufnahme des Videos geschwenkt wird.

Das erste und/oder die weiteren Bilder können aus einer ersten Teilmenge der Einzelbilder der Videosequenz entnommen sein, wobei die Einzelbilder der ersten Teilmenge aus kranialen Positionen relativ zu dem Fuß und/oder einer Position in Draufsicht auf einen Teil des Körperteils, etwa einen Vorderfuß, aufgenommen wurden. Aus kranialen Positionen und/oder Draufsicht aufgenommene Bilder eignen sich insbesondere zur Bestimmung einer Breite des Fußes.

Alternativ können das erste und/oder die weiteren Bilder aus einer zweiten Teilmenge der Einzelbilder der Videosequenz entnommen sein, wobei die Einzelbilder der zweiten Teilmenge aus kranial-lateralen Positionen relativ zu dem Fuß aufgenommen wurden. Aus kranial-lateralen Positionen aufgenommene Bilder eignen sich insbesondere zur Bestimmung einer Länge des Fußes.

Alternativ können das erste und/oder die weiteren Bilder aus einer dritten Teilmenge der Einzelbilder der Videosequenz entnommen sein, wobei die Einzelbilder der dritten Teilmenge aus lateralen Positionen relativ zu dem Fuß aufgenommen wurden. Aus lateralen Positionen aufgenommene Bilder eignen sich insbesondere zur Bestimmung einer Höhe des Fußes, beispielsweise einer Höhe des Fußes im Rist-Bereich.

Alternativ können das erste und/oder die weiteren Bilder aus einer vierten Teilmenge der Einzelbilder der Videosequenz entnommen werden, wobei Einzelbilder der vierten Teilmenge aus Positionen aufgenommen sind, in welcher eine Ferse des Fußes erkennbar ist, und/oder aus Positionen, in der mehrere Ecken des Referenzobjekts, beispielsweise wenigstens 3 Ecken oder 4 Ecken des Referenzobjekts, in dem jeweiligen Einzelbild erkennbar sind.

Das Bestimmen der weiteren segmentierten Bilder kann ähnlich wie das Bestimmen des ersten segmentierten Bildes durch Anwenden des konvolutionalen neuronalen Netzes auf die weiteren Bilder erfolgen. Darüber hinaus kann das Bestimmen der weiteren Eckpunkte der Grundfläche des Referenzobjekts in den weiteren segmentierten Bildern ähnlich wie das Bestimmen der ersten Eckpunkte der Grundfläche in dem ersten segmentierten Bild erfolgen. Beispielsweise können für jedes der weiteren segmentierten Bilder Kanten der Grundfläche des Referenzobjekts in dem segmentierten Bild bestimmt werden, und weitere Schnittpunkte der Kanten in den weiteren segmentierten Bildern können bestimmt werden. Die weiteren Eckpunkte der Grundfläche in den weiteren segmentierten Bildern können die weiteren Schnittpunkte oder eine verarbeitete/gefilterte Variante der weiteren Schnittpunkte sein.

Gemäß einer Ausführungsform umfasst das Verfahren ferner Laden wenigstens eines weiteren Bildes, wobei das wenigstens eine weitere Bild den Körperteil und das Referenzobjekt in einer weiteren Bildaufnahmerichtung zeigt, welche sich von der Bildaufnahmerichtung des ersten Bildes unterscheidet. Ferner umfass das Verfahren Bestimmen wenigstens eines weiteren segmentierten Bildes durch Anwenden des konvolutionalen neuronalen Netzes auf das wenigstens eine weitere Bild, und Überlagern wenigstens eines Teils des ersten segmentierten Bildes und wenigstens eines Teils des weiteren segmentierten Bildes unter Generieren eines zusammengesetzten segmentierten Bildes, wobei das anatomische Maß basierend auf dem zusammengesetzten segmentierten Bild bestimmt wird. In anderen Worten kann das Verfahren ferner ein Überlagern wenigstens eines Teils eines ersten segmentierten Bildes mit wenigstens einem Teil wenigstens eines weiteren segmentierten Bildes umfassen, beispielsweise unter Generieren eines zusammengesetzten segmentierten Bildes. Das Überlagern kann dabei ein Zusammenfügen des Teils des ersten segmentierten Bildes und des Teils des wenigstens einen weiteren segmentierten Bildes umfassen, so dass das Körperteil in dem zusammengesetzten segmentierten Bild nachgebildet ist. Dies kann eine genaue Bestimmung des anatomischen Maßes ermöglichen. Optional kann das erste segmentierte Bild und/oder das wenigstens eine weitere segmentierte Bild entzerrt sein.

In einem Beispiel kann der Teil des ersten segmentierten Bildes einen Vorderfuß (bzw. Vorfuß) abbilden und der Teil des wenigstens einen weiteren segmentierten Bildes kann einen Hinterfuß und/oder einen Fersenbereich abbilden, so dass in dem zusammengesetzten segmentierten Bild beispielsweise der gesamte Fuß abgebildet ist. Dies kann eine genaue Bestimmung eines oder mehrerer anatomischer Maße des Fußes ermöglichen. Insbesondere kann so eine Länge, eine Breite, eine Höhe, ein Umfangsmaß, ein des Fußes mit hoher Genauigkeit bestimmt werden. Ein Umfangsmaß, beispielsweise ein Fersenumfangsmaß und/oder ein Ballenumfangsmaß, kann dabei basierend auf einem oder mehreren zweidimensionalen Maßen, wie Breite, Höhe und Länge des Fußes, abgeschätzt werden. Üblicherweise kann die Breite eines Fußes rund 35%, beispielsweise zwischen 30% und 40%, des Ballenumfangsmaßes des Fußes betragen.

Gemäß einer Ausführungsform umfasst das Überlagern ferner ein Bestimmen und/oder Ermitteln einer Gesamtfläche, einer Länge und/oder einer Breite des Referenzobjekts in dem zusammengesetzten segmentierten Bild und ein Vergleichen der bestimmten Gesamtfläche, der Länge und/oder der Breite mit einem Referenzmaß des Referenzobjekts, beispielsweise einer Referenzfläche, einer Referenzbreite und/oder einer Referenzlänge. Auf diese Weise kann die Korrektheit des zusammengesetzten Bildes überprüft werden. Ist das Referenzobjekt beispielsweise ein DIN-A4-Blatt, so kann die ermittelte Länge des DIN-A4-Blattes in dem zusammengesetzten segmentierten Bild mit der Referenzlänge von 297mm verglichen werden. Alternativ oder zusätzlich kann die Breite und/oder die Fläche verwendet werden. Das Referenzmaß kann dabei in einem Speicher des Servers hinterlegt sein.

Optional kann die Korrektheit des zusammengesetzten segmentierten Bildes anhand einer Bestimmung einer Abweichung der ermittelten Gesamtfläche, der Länge und/oder der Breite von dem Referenzmaß des Referenzobjekts bestimmt werden. Die Abweichung kann beispielsweise mit einem vorbestimmten Schwellenwert verglichen werden. Bei Erreichen oder Überschreiten des Schwellenwerts kann das zusammengesetzte segmentierte Bild beispielsweise verworfen werden.

Gemäß einer Ausführungsform umfasst das Verfahren ferner ein Ermitteln eines Typs des Körperteils basierend auf einem segmentierten Bild, beispielsweise dem ersten segmentierten Bild und/oder einem weiteren segmentierten Bild. Der Typ des Körperteils kann beispielsweise ein linker oder rechter Fuß sein. Das Bestimmen des Typs kann beispielsweise ein Bestimmen einer Pixeldichte und/oder eines Pixelanteils von dem Körperteil zugeordneten Pixeln in dem segmentierten Bild und/oder in wenigstens einem vorbestimmten Bereich des segmentierten Bildes umfassen.

Alternativ oder zusätzlich kann das Bestimmen des Typs beispielsweise ein Bestimmen einer ersten Pixeldichte und/oder eines ersten Pixelanteils von dem Körperteil zugeordneten Pixeln in einem ersten vorbestimmten Bereich des segmentierten Bildes und ein Bestimmen einer zweiten Pixeldichte und/oder eines zweiten Pixelanteils von dem Körperteil zugeordneten Pixeln in einem zweiten vorbestimmten Bereich des segmentierten Bildes umfassen. Der erste und zweite Bereich können sich dabei voneinander unterscheiden. Die erste Pixeldichte und/oder der erste Pixelanteil können mit der zweiten Pixeldichte und/oder dem zweiten Pixelanteil verglichen werden, um den Typ des Körperteils zu bestimmen.

Am Beispiel der Bestimmung des linken bzw. rechten Fußes kann beispielsweise die erste Pixeldichte in einer linken Hälfte eines Bildbereichs bestimmt werden und die zweite Pixeldichte kann in einer rechten Hälfte des Bildbereiches bestimmt werden. Der Bildbereich kann dabei den Vorfuß abbilden, sodass anhand der unterschiedlichen Pixeldichten, welche durch die in den beiden Hälften aufgrund der dort erkennbaren (unterschiedlichen) Zehen hervorgerufen werden, festgestellt werden kann, ob es sich um den linken oder den rechten Fuß handelt.

Ferner können weitere entzerrte Bilder durch separates Entzerren von zumindest einem Teil der weiteren segmentierten Bilder unter Verwendung der weiteren Eckpunkte bestimmt werden. Wenn beispielsweise die ermittelten Eckpunkte der Grundfläche in einem der weiteren segmentierten Bilder unzuverlässig erscheinen, beispielsweise, weil sie stark abweichen von Eckpunkten der Grundfläche, die für andere segmentierte Bilder bestimmt wurden, so kann das segmentierte Bild mit den unzuverlässig erscheinenden Eckpunkten verworfen werden, d.h. für dieses segmentierte Bild wird kein entzerrtes Bild bestimmt. Das separate Entzerren von zumindest einem Teil der weiteren segmentierten Bilder ist vorzugsweise ein dem Entzerren des ersten segmentierten Bildes ähnliches perspektivisches Entzerren.

Für das erste entzerrte Bild und die weiteren entzerrten Bilder können nun separat Messwerte des anatomischen Maßes bestimmt werden. Das anatomische Maß kann dann auf der Grundlage der für das erste entzerrte Bild und die weiteren entzerrten Bilder separat bestimmten Messwerte bestimmt werden, beispielsweise durch eine Mittelung der separat bestimmten Messwerte.

Wenn die Kamera während der Aufnahme des Videos geschwenkt wird, können das erste Bild und die weiteren Bilder von unterschiedlichen Positionen und aus unterschiedlichen Richtungen aufgenommen worden sein. Dabei kann in einem der Bilder ein Schattenwurf abgebildet sein, der in einem anderen Bild aufgrund der unterschiedlichen Aufnahmeposition und Aufnahmerichtung nicht auftritt. Der Schattenwurf kann beispielsweise dazu führen, dass Pixel des Bildes von dem konvolutionalen neuronalen Netz fehlerhaft als Körperteil klassifiziert werden, sodass der für das entsprechende Bild bestimmte Messwert des anatomischen Maßes größer ist als das tatsächliche anatomische Maß. Das Bestimmen von Messwerten des anatomischen Maßes für mehrere entzerrte Bilder bietet den Vorteil, dass beispielsweise durch einen Schattenwurf gestörte Messwerte erkannt und entfernt werden können. Dazu können Messwerte unterschiedlicher entzerrter Bilder miteinander verglichen werden. Ferner kann detektiert werden, ob die für das erste entzerrte Bild und die weiteren entzerrten Bilder bestimmten Messwerte stark voneinander abweichen, insbesondere stärker als ein Schwellwert von beispielsweise 1 mm, 2 mm, 5 mm oder 10 mm. Sollte dies der Fall sein, kann das erfindungsgemäße Verfahren unter Verwendung anderer Einzelbilder des Videos wiederholt werden, oder die Videosequenz kann als für die Bestimmung des anatomischen Maßes unbrauchbar verworfen werden. Außerdem kann ein Cluster von nahe beieinanderliegenden Messwerten bestimmt werden, und das Endergebnis für das zu bestimmende anatomische Maß kann beispielsweise durch eine Mittelung von Messwerten des Clusters berechnet werden, wobei die Mittelung von Messwerten zu einer Reduktion der Standardabweichung und somit einer verbesserten Schätzgenauigkeit führen kann.

Gemäß einer weiteren Ausführungsform umfasst das Verfahren ferner das Laden weiterer Bilder, wobei die weiteren Bilder den Körperteil und das Referenzobjekt mit der bekannten Distanz in mehreren Bildaufnahmerichtungen zeigen. Wie voranstehend erläutert, können das erste Bild und/oder die weiteren Bilder beispielsweise Einzelbilder eines Videos sein. Das Video kann von einem Smartphone oder einem anderen Bediengerät mit Kamera, beispielsweise einem Tablet PC, einem Laptop, einem PC, einem Smartdevice oder dergleichen, aufgenommen worden sein.

Gemäß einer Ausführungsform umfasst das Verfahren ferner ein Veranlassen eines Bediengeräts, beispielsweise eines Smartphones, zur Ausgabe eines Hinweises und/oder Signals an einer Benutzerschnittstelle des Bediengeräts. Bei der Benutzerschnittstelle kann es sich beispielsweise um ein Display, einen Lautsprecher oder einen haptischen Signalgeber handeln. Der Hinweis und/oder das Signal kann insbesondere die Person anleiten, das Bediengerät derart um das Körperteil zu führen, dass ein für die Bestimmung des anatomischen Maßes geeigneter Satz von Bildern und/oder ein geeignetes Video aufgenommen wird. Alternativ oder zusätzlich kann der Hinweis und/oder das Signal der Person Feedback bzw. Rückmeldung darüber geben, ob bisher aufgenommene Bilder und/oder Teile des Videos für die Bestimmung des anatomischen Maßes geeignet sind. Der Hinweis und/oder das Signal kann dabei eine Textnachricht, ein optisches Signal ein akustisches Signal und/oder ein haptisches Signal sein.

Gemäß einer Ausführungsform umfasst das Verfahren ferner ein Anleiten, durch den Server, einer Person, das Bediengerät während der Aufnahme eines Videos um einen Winkelbereich um das Körperteil herum zu führen. Das Anleiten kann dabei durch Bereitstellen und/oder Übermitteln eines Hinweises und/oder Signals durch den Server an das Bediengerät erfolgen.

Gemäß einer Ausführungsform umfasst das Verfahren ferner ein Veranlassen des Bediengeräts durch den Server, etwa durch Übermitteln eines Steuersignals von dem Server an das Bediengerät, zur Aufnahme (und/oder zur Übermittlung an den Server) eines oder mehrerer Screenshots während das Bediengerät um das Körperteil geführt wird und/oder während der Aufnahme des Videos. Der wenigstens eine Screenshot kann ferner von dem Server analysiert werden, um festzustellen, ob das aufgenommene Video für die Bestimmung des anatomischen Maßes geeignet ist. Der wenigstens eine Screenshot kann dabei im Vergleich zu den Bildern des Videos in verringerter Auflösung vorliegen und dem Server bereitgestellt werden. Dies kann eine schnelle Rückmeldung durch den Server an das Bediengerät ermöglichen, insbesondere während das Video aufgenommen wird.

Anhand des wenigstens einen Screenshots kann der Server eines oder mehrere vordefinierte Kriterien zur Überprüfung einer Eignung des Videos zur Bestimmung des anatomischen Maßes prüfen. Beispielsweise kann zu Beginn der Videoaufnahme, ein Screenshot von dem Server analysiert werden, um dem Nutzer (bzw. der Person/dem Bediener des Bediengeräts) direkt bzw. in nahezu Echtzeit ein Feedback und/oder Rückmeldung zu geben. Beispielsweise kann der Server anhand des wenigstens einen Screenshots überprüfen, ob der Bildausschnitt zu klein oder zu groß, ob das Referenzobjekt gut erkennbar ist, ob mehrere Ecken des Referenzobjekts (beispielsweise drei oder vier Ecken) erkennbar sind, ob die Beleuchtungssituation gut genug, und/oder ob die Bilder ausreichende Schärfe aufweisen. Durch Analyse eines oder mehrerer Screenshots kann so eine vereinfachte Vorab-Analyse ermöglicht werden, um dem Nutzer ein Feedback und/oder Rückmeldung zu geben, damit er/sie seine Aufnahme noch optimieren kann und/oder ob die bisherige Aufnahme für die Bestimmung des anatomischen Maßes geeignet ist.

Nachfolgend ist dies in einem erläuternden Beispiel ausgeführt. Ein Nutzer kann mit dem Bediengerät beispielsweise ein Video von ca. 4s aufnehmen und erst nach Abschluss der Aufnahme kann das Video in maximal verfügbarer Auflösung (üblich Full-HD) an den Server gesendet werden. Während der Aufnahme des Videos kann der Server das Bediengerät zur Aufnahme und/oder Übermittlung eines oder mehrerer Screenshots eines oder mehrerer (Video)Bilder veranlassen, welche etwa in geringer Auflösung von z.B. 224x224 Pixel dem Server bereitgestellt und von diesem analysiert werden können. Der Server kann beispielsweise in einem Segmentation-Test anhand des wenigstens Screenshots ermitteln, ob ein Bild des Videos, welches dem Screenshot entspricht, ausreichend gut segmentierbar ist und/oder ob darauf der Körperteil und/oder das Referenzobjekt (etwa Fuß und/oder Papier) sichtbar sind. Alternativ oder zusätzlich kann der Server einen Blurriness-Test anhand des wenigstens einen Screenshots durchführen, um festzustellen, ob das Bild scharf genug ist und/oder ob eine Schärfe des Bildes in einem vordefinierten Bereich liegt. Alternativ oder zusätzlich kann der Server einen Contrast-Test anhand des wenigstens einen Screenshots durchführen, um festzustellen, ob die Beleuchtung des Bildes in einem vordefinierten Bereich liegt. Alternativ oder zusätzlich kann der Server einen Plausibilitätscheck anhand des wenigstens einen Screenshots durchführen, um festzustellen, ob ein Flächenanteil des Referenzobjekts, etwa eines Papiers, zwischen 20-75% des gesamten Bildes entspricht (z.B. in Draufsicht bis zu 75%), ob mehrere Ecken des Referenzobjekts, beispielsweise drei oder vier Ecken, erkennbar sind, und/oder ob sich das Körperteil (etwa der Fuß) innerhalb eines Rahmens befindet, der von Ecken des Referenzobjekts (z.B. drei oder vier Ecken) aufgespannt wird. Alternativ oder zusätzlich kann festgestellt werden, ob das Körperteil eine der Ecken verdeckt. Dem Nutzer kann dabei entsprechend den durchgeführten Tests und/oder den überprüften Kriterien ein oder mehrere Hinweise bzw. Signale gegeben werden, um die Aufnahme des Videos zu optimieren und/oder um eine Korrektheit des aufgenommenen Videos anzuzeigen.

Eine Analyse eines oder mehrerer Screenshots kann ein Feedback und/oder eine Rückmeldung an den Nutzer im Aufnahmeprozess innerhalb weniger hundert Millisekunden, beispielsweise rund 300 ms, ermöglichen. Dabei kann auf spezifische Fehlercodes, Hinweise und/oder Signale an den Nutzer zurückgegriffen werden, um dem Nutzer Hinweise zu Verbesserung seiner noch laufenden Messung zu geben.

Beispielsweise kann auf diese Weise eine Startposition der Aufnahme des Videos und optional eine Endposition der Aufnahme basierend auf entsprechenden Screenshots überprüft werden. Auch eine Überprüfung eines weiteren Screenshots an einer Position zwischen Startposition und Endposition ist möglich.

Der gleiche Prozess kann auch zum Finden und/oder Auswählen von geeigneten Bildern für die finale Bildauswertung bzw. die Bestimmung des anatomischen Maßes genutzt werden, beispielsweise um schnell (z.B. innerhalb 100 ms) Einzelbilder nach den voranstehend genannten Kriterien zu prüfen, etwa bevor mit voller verfügbarer Auflösung (z.B. Full-HD (1920x1080)) das anatomische Maß bestimmt wird.Gemäß einer weiteren Ausführungsform umfasst das Bestimmen des anatomischen Maßes das Bestimmen des Medians der Messwerte. Der Median der Messwerte ist ein robuster Schätzer für das zu bestimmende anatomische Maß, da Ausreißer unter den Messwerten den Median kaum beeinflussen.

In anderen Ausführungsformen können andere robuste Schätzer verwendet werden, beispielsweise Schätzer auf der Grundlage einer Clusteranalyse. Insbesondere kann der k-means Clustering Algorithmus angewandt werden, um k Clusterzentren zu bestimmen. Der Parameter k kann im Verhältnis zu der Zahl der Messwerte bestimmt werden, beispielsweise als die Hälfte, ein Drittel oder ein Viertel der Zahl der Messwerte. Nachfolgend kann das Clusterzentrum bestimmt werden, dem die meisten Messwerte zugeordnet sind. Das Endergebnis für das anatomische Maß kann als Mittelwert der diesem Cluster zugeordneten Messwerte bestimmt werden. Der derart bestimmte Schätzer für das anatomische Maß ist robust insbesondere im Hinblick auf eine unzureichende Ausleuchtung des Körperteils bei der Aufnahme der Bilder sowie im Hinblick auf verdeckte oder unscharf abgebildete Bildabschnitte. Ferner kann durch die Mittelung von Messwerten eine Verbesserung der Schätzgenauigkeit erreicht werden. Sollte die Zahl der Ausreißer geringer sein als k-1, so kann die Mittelung von Messwerten, die nahe beieinanderliegenden Clusterzentren zugeordnet sind, eine weitere Verbesserung der Genauigkeit des Schätzers ermöglichen.

Gemäß einer weiteren Ausführungsform umfasst das Bestimmen des anatomischen Maßes das Vergleichen von Messwerten. Insbesondere kann detektiert werden, ob Abweichungen zwischen den für die mehreren entzerrten Bilder bestimmten Messwerten größer als ein Schwellwert sind. Sollte dies der Fall sein, kann beispielsweise das erfindungsgemäße Verfahren unter Verwendung anderer Bilder wiederholt werden bis die Abweichungen zwischen den Messwerten kleiner oder gleich dem Schwellwert sind. Weiterhin kann einem Nutzer indiziert werden, dass das anatomische Maß unter Verwendung des ersten und der weiteren Bilder nicht zuverlässig bestimmt werden konnte.

Gemäß einer weiteren Ausführungsform umfasst das Verfahren ferner das Detektieren von Ausreißern unter den ersten und/oder den weiteren Eckpunkten der Grundfläche in dem ersten und/oder den weiteren segmentierten Bildern.

Das Detektieren von Ausreißern unter den ersten und/oder den weiteren Eckpunkten der Grundfläche kann insbesondere das Zuordnen eines Eckpunkts der Grundfläche in einem segmentierten Bild zu einem Eckpunkt der Grundfläche in einem anderen segmentierten Bild umfassen. Wenn beispielsweise das Referenzobjekt eine rechteckige Grundfläche aufweist, können die vier Eckpunkte der Grundfläche in einem segmentierten Bild bijektiv den vier Eckpunkten der Grundfläche in einem anderen segmentierten Bild zugeordnet werden. Eine derartige Zuordnung von Eckpunkten der Grundfläche kann für mehrere segmentierte Bilder erfolgen. Insbesondere wenn das erste Bild und die weiteren Bilder auf der Grundlage von Einzelbildern eines Videos bestimmt wurden, können Eckpunkte der Grundfläche in den segmentierten Bildern mittels eines Tracking-Algorithmus nachverfolgt werden, um Eckpunkte der Grundfläche in unterschiedlichen segmentierten Bildern zuzuordnen.

Danach können einander zugeordnete Eckpunkte der Grundfläche in unterschiedlichen segmentierten Bildern verglichen werden. Wenn beispielsweise zwei einander zugeordnete Eckpunkten der Grundfläche in unterschiedlichen segmentierten Bildern um mehr als einen Schwellwert voneinander abweichen, so kann dies bedeuten, dass für zumindest einen der zugeordneten Eckpunkte ein Ausreißer, d.h. ein stark fehlerbehafteter Schätzwert, vorliegt. Durch Vergleichen einander zugeordneter Eckpunkte der Grundfläche in mehreren segmentierten Bildern kann identifiziert werden, welche der ermittelten Eckpunkte Ausreißer sind.

Segmentierte Bilder, für die die ermittelten Eckpunkte der Grundfläche Ausreißer aufweisen, können verworfen werden, d.h. für diese Bilder wird beispielsweise keine Entzerrung ausgeführt. Weiterhin können zusätzliche Bilder geladen werden, wenn festgestellt wird, dass die ermittelten Eckpunkte der Grundfläche in einem oder mehreren segmentierten Bildern Ausreißer aufweisen.

Gemäß einer weiteren Ausführungsform ist eine Reihenfolge von Aufnahmezeitpunkten des ersten Bildes und der weiteren Bilder bekannt, und das Detektieren von Ausreißern unter den Eckpunkten der Grundfläche in den segmentierten Bildern umfasst das Prädizieren und/oder Retrodizieren von Eckpunkten der Grundfläche auf der Grundlage der Reihenfolge der Aufnahmezeitpunkte des ersten Bildes und der weiteren Bilder.

Die Reihenfolge der Aufnahmezeitpunkte des ersten Bildes und der weiteren Bilder kann insbesondere bekannt sein, wenn das erste Bild und die weiteren Bilder auf der Grundlage von Einzelbildern eines Videos bestimmt wurden. In diesem Fall können auch die zeitlichen Abstände zwischen den Aufnahmezeitpunkten des ersten Bildes und der weiteren Bilder bekannt sein.

Ausreißer können detektiert werden, indem Eckpunkte der Grundfläche auf der Grundlage der Reihenfolge der Aufnahmezeitpunkte des ersten Bildes und der weiteren Bilder prädiziert und/oder retrodiziert werden. Dazu können die prädizieren und/oder retrodizierten Eckpunkte der Grundfläche mit für den entsprechenden Aufnahmezeitpunkt ermittelten Eckpunkten der Grundfläche in einem der segmentierten Bilder verglichen werden. Dabei bezeichnet das Prädizieren von Eckpunkten das Schätzen bzw. Vorhersagen jüngerer Eckpunkte auf der Grundlage älterer Eckpunkte, während das Retrodizieren von Eckpunkten das Schätzen älterer Eckpunkte auf der Grundlage jüngerer Eckpunkte bezeichnet.

Sowohl das Prädizieren als auch das Retrodizieren von Eckpunkten kann das Zuordnen eines Eckpunkts der Grundfläche in einem segmentierten Bild zu einem Eckpunkt der Grundfläche in einem anderen segmentierten Bild umfassen. Dabei werden die Eckpunkte der Grundfläche in zwei unterschiedlichen segmentierten Bildern vorzugsweise bijektiv zugeordnet. Die Zuordnung von Eckpunkten der Grundfläche in unterschiedlichen segmentierten Bildern kann insbesondere bei auf der Grundlage von Einzelbildern eines Videos bestimmten Bildern unter Verwendung eines Tracking-Algorithmus erfolgen.

Ein Eckpunkt kann auf der Grundlage eines oder mehrerer zugeordneter älterer Eckpunkte prädiziert werden. Dabei kann ein lineares, affines, polynomielles oder trigonometrisches Modell für die zeitliche Veränderung der Eckpunkte der Grundfläche in den segmentierten Bildern verwendet werden. In ähnlicher Weise kann ein Eckpunkt auf der Grundlage eines oder mehrerer zugeordneter jüngerer Eckpunkte retrodiziert werden.

Gemäß einer weiteren Ausführungsform umfasst das Verfahren ferner das Verwerfen eines segmentierten Bildes, wenn die für das segmentierte Bild bestimmten Eckpunkte einen Ausreißer aufweisen.

Das Detektieren eines Ausreißers für einen Eckpunkt der Grundfläche des Referenzobjekts in einem segmentierten Bild indiziert, dass der ermittelte Eckpunkt stark fehlerbehaftet ist. Es ist zu erwarten, dass ein Entzerren des segmentierten Bildes auf der Grundlage des stark fehlerbehafteten Eckpunkts und das nachfolgende Bestimmen eines Messwertes für das anatomische Maß zu einem stark fehlerbehafteten Ergebnis führt. In diesem Fall kann das segmentierte Bild verworfen werden. Optional kann ein anderes segmentiertes Bild bestimmt werden, um das verworfene segmentierte Bild zu ersetzen, indem ein zusätzliches Bild geladen und mittels des konvolutionalen neuronalen Netzes segmentiert wird. Das zusätzlich geladene Bild kann einem früheren oder einem späteren Aufnahmezeitpunkt entsprechen im Vergleich zu dem verworfenen segmentierten Bild.

Gemäß einer weiteren Ausführungsform umfasst das Verfahren ferner das Bestimmen des ersten Bildes und der weiteren Bilder auf der Grundlage von nicht unmittelbar zeitlich aufeinander folgenden Einzelbildern eines Videos.

Während der Aufnahme des Videos kann die Kamera um den zu vermessenden Körperteil herumgeschwenkt werden. Dabei kann in einem Einzelbild des Videos für eine erste Aufnahmeposition und Aufnahmerichtung beispielsweise ein Schattenwurf auftreten, der in einem anderen Einzelbild für eine zweite Aufnahmeposition und Aufnahmerichtung nicht auftritt. Der Schattenwurf kann zu erheblichen Fehlern bei der Bestimmung des anatomischen Maßes führen. Durch das Verwenden nicht unmittelbar zeitlich aufeinander folgender Einzelbilder des Videos wird der Abstand zwischen den Aufnahmezeitpunkten der verwendeten Einzelbilder vergrößert und damit auch der Abstand zwischen den Aufnahmepositionen und Aufnahmerichtungen der verwendeten Einzelbilder, sodass es weniger wahrscheinlich ist, dass der Schattenwurf in allen verwendeten Einzelbildern auftritt. Beispielsweise durch das Verwerfen segmentierter Bilder, wenn die für das segmentierte Bild bestimmten Eckpunkte einen Ausreißer aufweisen, oder durch das Verwenden eines robusten Schätzers für das anatomische Maß, wie zum Beispiel des Medians der Messwerte, kann eine zuverlässige Bestimmung des anatomischen Maßes erfolgen, selbst wenn in einigen der verwendeten Einzelbildern ein Schattenwurf oder ein anderes Artefakt auftritt.

Gemäß einer weiteren Ausführungsform ist das anatomische Maß eine Länge, eine Breite oder eine Höhe eines menschlichen Fußes. Alternativ oder zusätzlich umfasst das Maß ein Umfangsmaß, ein Fersenumfangsmaß und/oder ein Ballenumfangsmaß des Fußes.

Das Bestimmen von Kleidungsstücken, die zur Anatomie und Körperkonstellation einer Person passen, kann insbesondere bei Schuhen mühsam und mit hohem Zeitaufwand verbunden sein. Bisher wurden Schuhgrößen mittels einer Längenangabe und teilweise einer Breitenangabe beschrieben. Da die dreidimensionale Form eines Fußes durch eine Längen- und eine Breitenangabe nur unzureichend beschrieben werden kann, führt die Auswahl eines Schuhs auf der Grundlage einer Längenangabe und evtl. einer Breitenangabe häufig zu einem schlecht passenden Schuh. Um das Bestimmen eines passenden Schuhs zu vereinfachen, können eine Länge, eine Breite und eine Höhe des Fußes bestimmt werden, und die bestimmten anatomischen Maße können mit entsprechenden Schuhinnenmaßen verglichen werden, um ein passendes Schuhmodell und eine passende Schuhgröße zu ermitteln. Insbesondere kann das Verfahren dazu ausgeführt sein, die Höhe des Fußes im Ristbereich bzw. im Bereich der Mittelfußknochen, der Keilbeine oder des Kahnbeins zu bestimmen. Es ist auch möglich, dass mehrere Längen, mehrere Breiten und/oder mehrere Höhen des Fußes bestimmt und mit entsprechenden Schuhinnenmaßen verglichen werden, um einen passenden Schuh zu bestimmen.

Gemäß einer weiteren Ausführungsform umfasst das Verfahren ferner das Bestimmen des ersten Bildes durch Reduktion einer Auflösung eines von einer digitalen Kamera aufgenommenen Bildes.

Die Auflösung des mit der Kamera aufgenommenen Bildes kann auf eine Auflösung reduziert werden, für die das konvolutionale neuronale Netz ausgeführt und insbesondere trainiert wurde.

Die Reduktion der Auflösung des mit der Kamera aufgenommenen Bildes kann zu einer Reduktion der Datenmenge des Bildes führen. Die Übertragung des ersten Bildes zu einer Datenverarbeitungsvorrichtung (zum Beispiel einem Server) zur Ausführung weiterer Verfahrensschritte benötigt dann weniger nachrichtentechnische Ressourcen (Zeitslots, Frequenzen und/oder Codes). Das erste Bild kann somit schneller und insbesondere in einem kürzeren Übertragungszeitraum zu der Datenverarbeitungsvorrichtung übertragen werden.

Die Reduktion der Auflösung des mit der Kamera aufgenommenen Bildes kann jedoch zu einer Reduktion der Entropie und des Informationsgehalts des Bildes führen. Dies kann zur Folge haben, dass der für das erste Bild bestimmte Messwert des anatomischen Maßes weniger genau ist.

Auch die weiteren Bilder können durch Reduktion der Auflösung von Bildern bestimmt werden, die von einer digitalen Kamera aufgenommen wurden. Durch die Reduktion der Auflösung können mehr Bilder pro Zeiteinheit zu einem Server übertragen werden, der weitere Verfahrensschritte wie das Segmentieren, das Bestimmen von Eckpunkten, das perspektivische Entzerren und/oder das Bestimmen des anatomischen Maßes ausführt. Die größere Zahl der dazu zur Verfügung stehenden Bilder verringert insbesondere die Wahrscheinlichkeit, dass alle Bilder störende Artefakte aufweisen, die zu erheblichen Fehlern bei der Bestimmung des anatomischen Maßes führen können. Ein solches Artefakt kann beispielsweise ein Schattenwurf sein oder ein verdeckter Bildabschnitt, der zur Bestimmung des anatomischen Maßes signifikant ist. Ein weiteres Artefakt kann ein unscharf abgebildeter Bildabschnitt sein, beispielsweise aufgrund eines für diesen Bildabschnitt ungeeigneten Kamerafokus. Beispielsweise durch das Verwerfen segmentierter Bilder, wenn die für das segmentierte Bild bestimmten Eckpunkte einen Ausreißer aufweisen, oder durch das Verwenden eines robusten Schätzers für das anatomische Maß, wie zum Beispiel des Medians der Messwerte, kann das anatomische Maß mit hoher Wahrscheinlichkeit zuverlässig bestimmt werden, selbst wenn in dem ersten Bild oder in einigen weiteren Bildern ein Schattenwurf oder ein anderes Artefakt auftritt. Darüber hinaus kann beispielsweise durch eine Mittelung von Messwerten die Standardabweichung des Endergebnisses für das zu bestimmende anatomische Maß reduziert werden. Die Reduktion der Auflösung kann somit zu einer verbesserten Schätzgenauigkeit des anatomischen Maßes führen, wenn die Reduktion der Auflösung mit einer Erhöhung der Zahl der Bilder einhergeht, die für das Bestimmen des anatomischen Maßes berücksichtigt werden.

Gemäß einer weiteren Ausführungsform umfasst das Verfahren ferner das Bestimmen einer Eignung einer Aufnahmerichtung des ersten Bildes relativ zu dem Körperteil für die Bestimmung des anatomischen Maßes auf der Grundlage der ersten Eckpunkte der Grundfläche des Referenzobjekts in dem ersten segmentierten Bild, auf der Grundlage der bekannten Abmessungen der Grundfläche sowie auf der Grundlage einer bekannten Anordnung des Körperteils relativ zu der Grundfläche.

Auf der Grundlage der ersten Eckpunkte der Grundfläche des Referenzobjekts in dem ersten segmentierten Bild und den bekannten Abmessungen der Grundfläche kann eine Position und eine Orientierung der Kamera bei der Aufnahme des ersten Bildes relativ zu der Grundfläche des Referenzobjekts bestimmt werden. Wenn die Anordnung des Körperteils relativ zu der Grundfläche des Referenzobjekts bekannt ist, so kann die Richtung bestimmt werden, aus der der Körperteil in dem ersten Bild aufgenommen wurde.

Die Eignung der Aufnahmerichtung des ersten Bildes relativ zu dem Körperteil kann auch auf der Grundlage einer Transformationsmatrix, die zur perspektivischen Entzerrung des ersten Bildes verwendet werden kann, bestimmt werden.

Wenn beispielsweise bekannt ist, dass das Referenzobjekt ein DIN-A4-formatiges Blatt Papier ist, so kann auf der Grundlage der ersten Eckpunkte des Blatts Papier in dem ersten segmentierten Bild und den bekannten Abmessungen des DIN-A4-Formats eine Position und eine Orientierung der Kamera bei der Aufnahme des ersten Bildes relativ zu dem Blatt Papier bestimmt werden. Wenn ferner bekannt ist, dass der zu vermessende Körperteil ein Fuß ist, der auf dem DIN-A4-formatigen Blatt Papier steht, d.h. wenn das Blatt Papier parallel zu der Transversalebene der Person angeordnet ist, so kann auf der Grundlage dieser Information die Richtung bestimmt werden, aus der der Fuß in dem ersten Bild abgebildet ist.

Aus kranialer Richtung aufgenommene Bilder sind insbesondere geeignet zur Bestimmung einer Breite des Fußes. Aus kranial-lateraler Richtung aufgenommene Bilder sind insbesondere geeignet zur Bestimmung einer Länge des Fußes. Aus lateraler Richtung aufgenommene Bilder sind insbesondere geeignet zur Bestimmung einer Höhe des Fußes. Aus kranial-lateraler Richtung aufgenommene Bilder mit Sichtbarkeit der Ferse kann insbesondere ermöglichen, mehrere Bilder zu überlagern, so dass das zusammengesetzte Bild die Zehen und die Ferse abdeckt, was eine exakte Bestimmung der Fußlänge ermöglichen kann. Wenn beispielsweise bestimmt wurde, dass der Fuß in dem ersten Bild aus lateraler Richtung aufgenommen wurde und das zu bestimmende anatomische Maß eine Breite des Fußes ist, so kann das erste Bild als für die Bestimmung des anatomischen Maßes ungeeignet klassifiziert und/oder verworfen werden.

Wenn beispielsweise ein Video oder eine sonstige Vielzahl von Einzelbildern geladen wurde, um ein anatomisches Maß zu bestimmen, so kann für jedes der Einzelbilder bestimmt werden, ob es sich für die Bestimmung des anatomischen Maßes eignet. Dies ist insbesondere dann sinnvoll, wenn die Kamera während der Aufnahme des Videos bzw. der Aufnahme der Sequenz von Einzelbildern um den Körperteil herumgeschwenkt wurde. Ferner kann, wenn mehrere anatomische Maße des Körperteils zu bestimmen sind, für jedes der Einzelbilder bestimmt werden, welches anatomische Maß bzw. welche anatomischen Maße auf der Grundlage des Einzelbilds bestimmt werden können.

Gemäß einer weiteren Ausführungsform umfasst das Verfahren ferner das Bestimmen eines dreidimensionalen Modells eines Körperteils unter Verwendung mehrerer Bilder. Dies kann insbesondere das Zuordnen eines Eckpunkts der Grundfläche in einem segmentierten Bild zu einem Eckpunkt der Grundfläche in einem anderen segmentierten Bild umfassen. Wenn beispielsweise das Referenzobjekt eine rechteckige Grundfläche aufweist, können die vier Eckpunkte der Grundfläche in einem segmentierten Bild bijektiv den vier Eckpunkten der Grundfläche in einem anderen segmentierten Bild zugeordnet werden. Eine derartige Zuordnung von Eckpunkten der Grundfläche kann für mehrere segmentierte Bilder erfolgen. Insbesondere wenn das erste Bild und die weiteren Bilder auf der Grundlage von Einzelbildern eines Videos bestimmt wurden, können Eckpunkte der Grundfläche in den segmentierten Bildern mittels eines Tracking-Algorithmus nachverfolgt werden, um Eckpunkte der Grundfläche in unterschiedlichen segmentierten Bildern zuzuordnen.

Gemäß einer weiteren Ausführungsform umfasst ein Verfahren zur Bestimmung eines Schuhmodells und einer Schuhgröße das Bestimmen von Schuhinnenlängen, Schuhinnenbreiten und/oder Schuhinnenhöhen für eine Vielzahl von Schuhmodellen und Schuhgrößen, das Speichern der Schuhinnenlängen, Schuhinnenbreiten und/oder Schuhinnenhöhen in einer Datenbank, das Bestimmen einer oder mehrerer Längen, einer oder mehrerer Breiten und/oder einer oder mehrerer Höhen eines Fußes gemäß dem dargelegten Verfahren, und das Durchsuchen der Datenbank unter Berücksichtigung der einen oder mehreren Längen, der einen oder mehreren Breiten und/oder der einen oder mehreren Höhen des Fußes und/oder eines oder mehrerer Umfangsmaße des Fußes (z.B. Fersenumfangsmaß und/oder Ballenumfangsmaß), um ein Schuhmodell und eine Schuhgröße zu bestimmen.

Jeder menschliche Körperteil ist bezüglich seiner Proportionen einzigartig und der Mensch muss aus einer Auswahl von endlich vielen Konfektionsmodeartikeln passende Bekleidung finden, die ihm gefällt und ergonomisch zu ihm passt. Insbesondere Schuhgrößen sind bisher nicht herstellerübergreifend genormt, sodass schon die Schuhinnenlänge, die über die Schuhgröße angegeben wird, nicht zur eindeutigen Größenbestimmung verwendet werden kann. Die Breite der Schuhe und die Höhe im Rist-Bereich werden durch heutige Schuhgrößen nicht oder nur ansatzweise beschrieben. Zusätzliche Ungenauigkeiten und/oder Ungewissheiten können durch die Umrechnung von EU, US und UK Größen entstehen.

Um den Kauf eines passenden Schuhs zu vereinfachen, werden zunächst ein oder mehrere anatomische Maße des Fußes bestimmt. Insbesondere können eine oder mehrere Längen, eine oder mehrere Breiten und/oder eine oder mehrere Höhen des Fußes bestimmt werden. Vorzugsweise werden das anatomische Maß oder die anatomischen Maße des Fußes mit dem erfindungsgemäßen Verfahren unter Verwendung eines konvolutionalen neuronalen Netzes bestimmt, es können aber auch andere Verfahren verwendet werden. Als Ergebnis liegen ein oder mehrere anatomische Maße oder sogar ein dreidimensionales Modell des Fußes vor.

Damit ein passender Schuh zugeordnet werden kann, werden Schuhinnenlängen, Schuhinnenbreiten und/oder Schuhinnenhöhen für eine Vielzahl von Schuhmodellen und Schuhgrößen bestimmt. Das Bestimmen der Schuhinnenlängen, Schuhinnenbreiten und/oder Schuhinnenhöhen kann das Messen dieser Schuhinnenmaße umfassen. Es ist aber auch möglich, dass die Schuhinnenlängen, Schuhinnenbreiten und/oder Schuhinnenhöhen von Schuhherstellern, Händlern oder sonstigen Dritten gemessen werden und dass das Bestimmen der Schuhinnenlängen, Schuhinnenbreiten und/oder Schuhinnenhöhen das Zugreifen auf von Schuhherstellern, Händlern oder sonstigen Dritten gemessene Schuhinnenmaße umfasst. Ferner können Schuhinnenlängen, Schuhinnenbreiten und/oder Schuhinnenhöhen auf der Grundlage von Maßen der in den Schuhen verwendeten Leisten bestimmt werden. Es können auch mehrere Schuhe desselben Modells und derselben Schuhgröße vermessen werden, um herstellungsbedingte Varianzen der Schuhinnenmaße berücksichtigen zu können.

Für die Vermessung der Schuhe kann ein geeignetes Messsystem verwendet werden. Die Vermessung kann auf kameraoptischen Sensoren, Laserscannern, Computertomografie oder auf taktilen Sensoren beruhen. Dabei können Schuhe für die Vermessung durch ein Spannsystem oder einen aufblasbaren Fuß-Imitator in Form gebracht werden. Die gemessenen Schuhinnenmaße werden sodann in einer Datenbank gespeichert. Bei Stretch-Textilien oder anderen nachgiebigen Materialien kann auch die Dehnbarkeitsrate zusätzlich erfasst und gespeichert werden, um eine möglichst gute Bestimmung eines passenden Schuhs zu ermöglichen.

Die Datenbank kann beispielsweise auf einem Server gespeichert werden. Die Datenbank kann durchsucht werden, um ein passendes Schuhmodell und eine passende Schuhgröße zu bestimmen. Dazu können die ermittelten anatomischen Maße des Fußes mit den Schuhinnenmaßen der vermessenen Schuhe verglichen werden. Dabei kann auf die Fußlänge eine Längenzugabe addiert werden, um eine ausreichende Bewegungsfreiheit des Fußes im Schuh zu gewährleisten. In ähnlicher Weise kann auf die Fußbreite eine Breitenzugabe und/oder auf die Fußhöhe eine Höhenzugabe addiert werden, um eine ausreichende Bewegungsfreiheit des Fußes im Schuh und gleichzeitig seinen guten Sitz zu gewährleisten. Die Längen-, Breiten- und/oder Höhenzugaben können vom Schuhmaterial abhängen (beispielsweise größere Werte bei einem Lederschuh als bei einem nachgiebigen Textilschuh). Das Durchsuchen der Datenbank, um ein passendes Schuhmodell und eine passende Schuhgröße zu bestimmen, kann auch auf der Grundlage eines statistischen Modells erfolgen.

Die anatomischen Maße der beiden Füße eines Menschen können sich signifikant voneinander unterscheiden. Die beiden Füße werden daher vorzugsweise separat vermessen, beispielsweise mit dem erfindungsgemäßen Verfahren zur Bestimmung anatomischer Maße. Ferner kann für anatomische Maße bestimmt und/oder gespeichert werden, ob sie dem linken oder dem rechten Fuß des Menschen zuzuordnen sind. Auf der Grundlage der anatomischen Maße der beiden Füße kann durch Durchsuchen der Datenbank ein Schuhmodell bestimmt werden, für das passende Schuhgrößen für beide Füße vorliegen. Dabei können im Ergebnis unterschiedliche Schuhgrößen für die beiden Füße bestimmt werden. Das Verfahren kann auch dazu ausgeführt sein, unterschiedliche Schuhmodelle für die beiden Füße zu erlauben, wobei dies in den meisten Fällen nicht gewünscht ist.

Ein dritter Aspekt der Erfindung betrifft ein Datenverarbeitungssystem umfassend Mittel zur Ausführung des erfindungsgemäßen Verfahrens.

Das Datenverarbeitungssystem kann beispielsweise ein Smartphone, ein Phablet, ein Tablet, ein Notebook, ein Desktop-Rechner oder ein Server sein. Das Datenverarbeitungssystem umfasst einen Prozessor, vorzugsweise einen Mikroprozessor. Ferner umfasst das Datenverarbeitungssystem eine oder mehrere Speichereinheiten, vorzugsweise zumindest eine nichtflüchtige Speichereinheit. Das Datenverarbeitungssystem kann auch mehrere Datenverarbeitungsvorrichtungen und Datenübertragungsvorrichtungen umfassen, wobei die mehreren Datenverarbeitungsvorrichtungen dazu ausgeführt sind, einzelne Schritte des erfindungsgemäßen Verfahrens auszuführen. Beispielsweise kann ein Smartphone dazu ausgeführt sein, das erste Bild und gegebenenfalls die weiteren Bilder auf der Grundlage von Einzelbildern eines mit der Smartphone-Kamera aufgenommenen Videos zu bestimmen. Das Smartphone kann das erste Bild und gegebenenfalls die weiteren Bilder mittels einer Kommunikationseinheit beispielsweise an einen Server übertragen, der weitere Schritte des erfindungsgemäßen Verfahrens ausführt.

Ein vierter Aspekt der Erfindung betrifft ein Computerprogramm, umfassend Befehle, die bei der Ausführung des Programms durch das Datenverarbeitungssystem dieses veranlassen, das erfindungsgemäße Verfahren auszuführen.

Ein fünfter Aspekt der Erfindung betrifft ein computerlesbares Speichermedium, auf dem das erfindungsgemäße Computerprogramm gespeichert ist.

Im Folgenden werden Ausführungsbeispiele der Erfindung unter Bezugnahme auf die Figuren beschrieben. Darin bezeichnen gleiche Bezugszeichen gleiche oder ähnliche Elemente. Gleiche oder ähnliche Elemente können aber auch durch unterschiedliche Bezugszeichen bezeichnet sein.

### KURZE BESCHREIBUNG DER FIGUREN

Figur 1 veranschaulicht die Aufnahme eines Videos zur Bestimmung von Maßen eines Fußes.
Figur 2 veranschaulicht ein Verfahren zur Bestimmung eines anatomischen Maßes auf der Grundlage von Einzelbildern eines Videos.
Figur 3 veranschaulicht ein Verfahren zur Bestimmung eines Schuhmodells und einer Schuhgröße.
Figuren 4a bis 4d zeigen jeweils ein segmentiertes Bild zur Illustration von Schritten des Verfahrens zur Bestimmung eines anatomischen Maßes.

### DETAILLIERTE BESCHREIBUNG VON AUSFÜHRUNGSBEISPIELEN

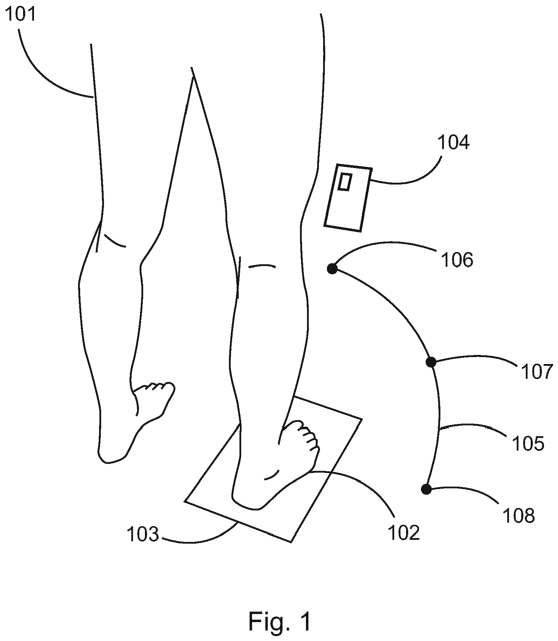

Die Figur 1 veranschaulicht die Aufnahme eines Videos zur Bestimmung anatomischer Maße eines Fußes 102 eines Menschen 101. Der Fuß 102 steht auf einem Referenzobjekt 103. Das Referenzobjekt 103 kann eine rechteckige Grundfläche mit bekannten Abmessungen aufweisen. Insbesondere kann das Referenzobjekt ein DIN-A4-formatiges Blatt Papier sein. Das Video kann beispielsweise mit der Videokamera eines Smartphones 104 aufgenommen werden.

Um beispielsweise eine Länge, eine Breite und eine Höhe (und/oder ein Umfangsmaß, wie Fersenumfangsmaß oder Ballenumfangsmaß) des Fußes 102 zu bestimmen, kann das Smartphone auf einem Viertelkreisbogen 105 um den zu vermessenden Fuß herumgeschwenkt werden. Dabei wird das Smartphone vorzugsweise in der Frontalebene der Person geführt, deren Fuß zu vermessen ist. Der Viertelkreisbogen kann einen kranial zu dem abzubildenden Fuß liegenden Punkt 106 mit einem lateral zu dem abzubildenden Fuß liegenden Punkt 108 verbinden. Während der Aufnahme des Videos bleibt die Blickrichtung der Smartphone-kamera vorzugsweise auf den Fuß gerichtet.

Die Führung entlang eines Viertelkreisbogens um den Fuß herum muss nicht genau eingehalten werden und dient allenfalls als grobe Richtlinie. Die Aufnahme des Videos kann vereinfacht werden, wenn die Kamera von einer dritten Person um den zu vermessenden Fuß herumgeschwenkt wird.

Das Video kann 20, 50, 100, 200 oder mehr Einzelbilder umfassen. Da das Smartphone während der Aufnahme des Videos geschwenkt wird, unterscheiden sich die Aufnahmepositionen und -richtungen der Einzelbilder.

Eine erste Teilmenge von Einzelbildern kann von Positionen aufgenommen worden sein, die in der Nähe des kranial relativ zu dem zu vermessenden Fuß 102 liegenden Punkt 106 liegen. Die Einzelbilder der ersten Teilmenge eignen sich insbesondere zur Bestimmung einer Breite des Fußes. Die Breite des Fußes kann somit bestimmt werden unter Verwendung eines oder mehrerer Einzelbilder der ersten Teilmenge.

Eine zweite Teilmenge von Einzelbildern kann von Positionen aufgenommen worden sein, die in der Nähe des kranial-lateral relativ zu dem zu vermessenden Fuß 102 liegenden Punkt 107 liegen. Die Einzelbilder der zweiten Teilmenge eignen sich insbesondere zur Bestimmung einer Länge des Fußes. Die Länge des Fußes kann somit bestimmt werden unter Verwendung eines oder mehrerer Einzelbilder der zweiten Teilmenge.

Eine dritte Teilmenge von Einzelbildern kann von Positionen aufgenommen worden sein, die in der Nähe des lateral relativ zu dem zu vermessenden Fuß 102 liegenden Punkt 108 liegen. Die Einzelbilder der dritten Teilmenge eignen sich insbesondere zur Bestimmung einer Höhe des Fußes, beispielsweise einer Höhe des Fußes im Rist-Bereich. Die Höhe des Fußes kann somit bestimmt werden unter Verwendung eines oder mehrerer Einzelbilder der dritten Teilmenge.

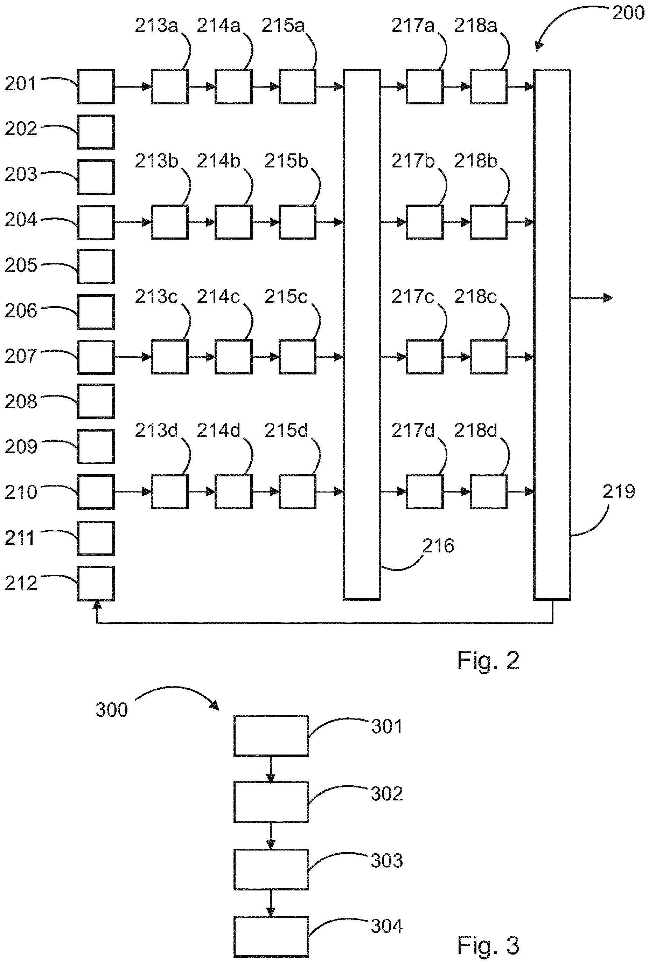

Figur 2 veranschaulicht ein Verfahren 200 zur Bestimmung eines anatomischen Maßes auf der Grundlage einer Sequenz von Einzelbildern 201 bis 212 eines Videos. Die Einzelbilder zeigen vorzugsweise einen Körperteil oder den gesamten Körper eines Menschen. Ferner zeigen die Einzelbilder ein Referenzobjekt mit bekannten Abmessungen, wobei eine Distanz des Referenzobjekts zu dem Körperteil in der Bildaufnahmerichtung der Bilder bekannt ist. Das Referenzobjekt weist vorzugsweise eine polygonale Grundfläche auf, wobei die Kanten der polygonalen Grundfläche vorzugsweise in den Einzelbildern zumindest teilweise abgebildet sind. Beispielsweise kann es sich bei dem Referenzobjekt um ein DIN-A4-formatiges Blatt Papier handeln, und auf diesem Papier kann ein Fuß stehen. Bei dem anatomischen Maß kann es sich beispielsweise um eine Länge, eine Breite oder eine Höhe des Fußes handeln. Alternativ oder zusätzlich kann es sich um ein Umfangsmaß, etwa ein Fersenumfangsmaß und/oder ein Ballenumfangsmaß, handeln.

In Figur 2 werden die Einzelbilder 201, 204, 207 und 210 verwendet, um das anatomische Maß zu bestimmen. In diesem Beispiel wird somit nur jedes dritte Einzelbild der Videosequenz zur Bestimmung des anatomischen Maßes verwendet. Wenn die Kamera während der Aufnahme des Videos geschwenkt wird, führt das Verwenden nicht unmittelbar aufeinanderfolgender Einzelbilder des Videos zu größeren Abständen zwischen den Aufnahmepositionen der Einzelbilder, sodass die Wahrscheinlichkeit reduziert wird, dass alle verwendeten Einzelbilder aus ungünstigen Positionen aufgenommen wurden. Dabei kann eine ungünstige Aufnahmeposition beispielsweise eine Aufnahmeposition sein, in der Schattenwürfe auftreten oder signifikante Bildabschnitte verdeckt sind.

In den optionalen Schritten 213a bis 213d kann die Auflösung der verwendeten Einzelbilder 201, 204, 207 und 210 reduziert werden. Die Reduktion der Auflösung der verwendeten Einzelbilder kann zu einer Reduktion der Datenmenge führen. Durch die Reduktion der Datenmenge kann erreicht werden, dass mehr Einzelbilder von der das Video aufnehmenden Vorrichtung an eine die weiteren Schritte des Verfahrens zur Bestimmung des anatomischen Maßes ausführenden Datenverarbeitungsvorrichtung gesendet werden können. Das Senden von mehr Einzelbildern kann beispielsweise den Vorteil haben, dass die verwendeten Einzelbilder einem größeren Bereich von Aufnahmepositionen entsprechen, sodass die Wahrscheinlichkeit reduziert werden kann, dass alle verwendeten Einzelbilder beispielsweise durch einen Schattenwurf, durch einen verdeckten Bildabschnitt oder durch einen unscharf abgebildeten Bildabschnitt für die präzise Bestimmung des anatomischen Maßes ungeeignet sind.

In den Schritten 214a bis 214d erfolgt eine semantische Segmentierung der verwendeten Einzelbilder mittels eines konvolutionalen neuronalen Netzes. Vorzugsweise wird dasselbe konvolutionale neuronale Netz separat auf jedes der verwendeten Einzelbilder angewandt, um die segmentierten Bilder zu bestimmen. Das konvolutionale neuronale Netz kann dazu ausgeführt sein, in den verwendeten Einzelbildern Körperteile, das Referenzobjekt und sonstige Bildabschnitte voneinander zu unterscheiden. Beispielsweise kann das konvolutionale neuronale Netz dazu ausgeführt sein, in einem Bild einen Fuß, das Referenzobjekt sowie den Fußboden voneinander zu unterscheiden. Möglicherweise ist das konvolutionale neuronale Netz dazu ausgeführt, weitere Objektklassen voneinander zu unterscheiden.

Die segmentierten Bilder können validiert werden (nicht in Figur 2 veranschaulicht). Wenn beispielsweise das Referenzobjekt eine rechteckförmige Grundfläche aufweist, und ferner bekannt ist, dass ein zu vermessender Fuß bei der Aufnahme des Videos mittig auf der Grundfläche steht, so unterbrechen Körperteile (Fuß oder Bein) den rechteckförmigen Umriss der Grundfläche des Referenzobjekts in den Bildern an höchstens einer Stelle. Beispielsweise aufgrund eines Schattenwurfs kann es jedoch sein, dass der rechteckförmige Umriss der Grundfläche in einem mit dem konvolutionalen neuronalen Netz bestimmten segmentierten Bild an mehreren Stellen von als Körperteil klassifizierten Bildabschnitten unterbrochen ist. Dies kann als Fehler bei der Bestimmung des ersten segmentierten Bildes detektiert werden, infolgedessen das segmentierte Bild verworfen werden kann. Weiterhin können zusätzliche Bilder geladen und segmentiert werden, um das verworfene segmentierte Bild zu ersetzen.

In den Schritten 215a bis 215d werden Eckpunkte der Grundfläche des Referenzobjekts in den segmentierten Bildern bestimmt. Die Eckpunkte der Grundfläche in den segmentierten Bildern können mittels zahlreicher Verfahren bestimmt werden, beispielsweise mit dem Harris-Eckpunkt-Detektor.

Wenn die Grundfläche des Referenzobjekts die Form eines Parallelogramms und vorzugsweise die Form eines Rechtecks aufweist, können die Eckpunkte der Grundfläche in den segmentierten Bildern bestimmt werden, indem die Kanten der Grundfläche in den segmentierten Bildern bestimmt werden. Die derart bestimmten Kanten der Grundfläche können jeweils einem von zwei Parallelenpaaren zugeordnet werden, und die Schnittpunkte der Kanten der Grundfläche können bestimmt werden. Die Schnittpunkte der Kanten können weiterverarbeitet werden. Beispielsweise können die Schnittpunkte der Kanten zeitlich gefiltert werden. Die Veränderung der Schnittpunkte über die Zeit kann zum Beispiel mittels einer affinen Funktion modelliert und eine Regression ausgeführt werden, um unter der Annahme zeitlich unkorrelierter Fehler bei der Bestimmung der Schnittpunkte die Fehler zu reduzieren. Die Eckpunkte der Grundfläche in den segmentierten Bildern können dann die Schnittpunkte oder die weiterverarbeiteten Schnittpunkte der Kanten der Grundfläche in den segmentierten Bildern sein.

In dem optionalen Schritt 216 kann eine Detektion von Ausreißern unter den Eckpunkten der Grundfläche in den segmentierten Bildern erfolgen. Dazu können die Eckpunkte der Grundfläche in den segmentierten Bildern nachverfolgt (getrackt) werden. Durch die Nachverfolgung von Eckpunkten der Grundfläche in den segmentierten Bildern über die Zeit wird eine Zuordnung eines Eckpunkts der Grundfläche in einem segmentierten Bild zu einem Eckpunkt der Grundfläche in einem anderen segmentierten Bild bestimmt. Wenn einander zugeordnete Eckpunkte der Grundfläche in unterschiedlichen segmentierten Bildern stark voneinander abweichen, so ist dies ein Indiz für das Vorliegen eines Ausreißers. Beispielsweise kann unter Berücksichtigung von Eckpunkten der Grundfläche in mehr als zwei segmentierten Bildern festgestellt werden, für welches der segmentierten Bilder die Eckpunkte einen Ausreißer aufweisen.

Wenn beispielsweise die Eckpunkte des auf dem Einzelbild 207 beruhenden segmentierten Bildes einen Ausreißer aufweisen, so könnte dieses segmentierte Bild verworfen werden, d.h. es würden keine weiteren Verarbeitungsschritte für das segmentierte Bild ausgeführt (nicht in Figur 2 veranschaulicht). Ferner kann ein zusätzliches Bild geladen und segmentiert werden, um das verworfene segmentierte Bild zu ersetzen. Wenn beispielsweise das in Schritt 214c bestimmte segmentierte Bild verworfen wird, so könnte das Einzelbild 206 oder das Einzelbild 208 geladen und segmentiert werden, um das verworfene segmentierte Bild zu ersetzen. Es könnten auch andere Einzelbilder des Videos geladen und segmentiert werden, um das verworfene segmentierte Bild zu ersetzen, einschließlich Bilder, die zeitlich vor dem Einzelbild 201 oder nach dem Einzelbild 212 aufgenommen wurden.

In den Schritten 217a bis 217d werden die in den Schritten 214a bis 214d bestimmten segmentierten Bilder unter Verwendung der in den Schritten 215a bis 215d bestimmten Eckpunkte der Grundfläche entzerrt. Dabei kann es sich um eine perspektivische Entzerrung handeln, sodass beispielsweise parallele Kanten der Grundfläche des Referenzobjekts in den entzerrten Bildern parallel verlaufen. Wenn das Referenzobjekt eine rechteckige Grundfläche aufweist, können die Schritte 217a bis 217d ferner ein Beschränken auf einen der rechteckigen Form der Grundfläche entsprechenden Bildausschnitt umfassen.

In den Schritten 218a bis 218d werden Messwerte für das anatomische Maß auf der Grundlage der entzerrten Bilder bestimmt.

Beispielsweise kann ein Messwert für die Breite eines Fußes bestimmt werden, indem in einem aus kranialer Position aufgenommenen Bild nach der Segmentierung und der perspektivischen Entzerrung ein distaler Abschnitt des Fußes bestimmt wird. Der distale Abschnitt des Fußes kann beispielsweise die distalen 40%, 50% oder 60% des Fußes umfassen. Nachfolgend kann ein Rechteck mit minimaler Fläche bestimmt werden, welches den distalen Abschnitt des Fußes vollständig umgibt. Der Messwert für die Breite des Fußes kann bestimmt werden als die Länge der kürzeren Seite des derart bestimmten Rechtecks.

In dem Schritt 219 können Messwerte für das anatomische Maß verglichen werden. Wenn beispielsweise Abweichungen zwischen Messwerten größer sind als ein Schwellwert, so kann eine Detektion von Ausreißern unter den Messwerten erfolgen. Ferner können Ausreißer unter den Messwerten verworfen werden. Der Schwellwert kann beispielsweise 1 mm, 2 mm, 5 mm oder 10 mm betragen. Die Rückkopplungsschleife in Figur 2 veranschaulicht, dass bei großen Abweichungen zwischen den Messwerten zusätzliche Einzelbilder des Videos geladen, segmentiert und entzerrt werden können, um zusätzliche Messwerte für das anatomische Maß zu bestimmen. Dadurch kann eine zuverlässigere Bestimmung des Endergebnisses für das anatomische Maß erreicht werden.

Insbesondere wenn die Abweichungen zwischen den Messwerten für das anatomische Maß wiederholt den Schwellwert überschreiten, kann das Verfahren abgebrochen werden, und/oder einem Nutzer kann indiziert werden, dass das Video nicht zur zuverlässigen Bestimmung des anatomischen Maßes geeignet ist. Anderenfalls kann das Endergebnis für das anatomische Maß beispielsweise als der Median der Messwerte bestimmt werden. Es ist aber auch möglich, dass andere robuste Schätzer für die Berechnung des anatomischen Maßes auf der Grundlage der Messwerte verwendet werden.

Figur 3 veranschaulicht ein Verfahren 300 zur Bestimmung eines Schuhmodells und einer Schuhgröße. Das Verfahren 300 umfasst das Bestimmen 301 von Schuhinnenlängen, Schuhinnenbreiten und/oder Schuhinnenhöhen für eine Vielzahl von Schuhmodellen und Schuhgrößen. Dies kann beispielsweise dadurch erfolgen, dass die Schuhinnenmaße gemessen werden oder dass zugegriffen wird auf von Herstellern, Händlern oder sonstigen Dritten gemessene Schuhinnenmaße. Ferner umfasst das Verfahren 300 das Speichern 302 der Schuhinnenlängen, Schuhinnenbreiten und/oder Schuhinnenhöhen in einer Datenbank. Weiterhin umfasst das Verfahren das Bestimmen 303 einer oder mehrerer Längen, einer oder mehrerer Breiten und/oder einer oder mehrerer Höhen eines Fußes gemäß dem erfindungsgemäßen oder einem anderen Verfahren zur Bestimmung anatomischer Maße. Darüber hinaus umfasst das Verfahren das Durchsuchen 304 der Datenbank unter Berücksichtigung der einen oder mehreren Längen, der einen oder mehreren Breiten und/oder der einen oder mehreren Höhen des Fußes, um ein Schuhmodell und eine Schuhgröße zu bestimmen.

Insbesondere Schuhgrößen sind bisher nicht herstellerübergreifend genormt, sodass schon die Schuhinnenlänge, die bisher über die Schuhgröße angegeben wird, nicht zur eindeutigen Größenbestimmung verwendet werden kann. Die durch die fehlende Normierung von Schuhgrößen auftretenden Varianzen können vermieden werden, indem sowohl die Schuhinnenmaße als auch die anatomischen Maße des Fußes vermessen werden, insbesondere die Schuhinnenlänge und die Fußlänge.

Die Breite der Schuhe und die Höhe im Rist-Bereich werden durch heutige Schuhgrößen nicht oder nur ansatzweise beschrieben. Um diese Unsicherheit zu vermeiden, werden vorzugsweise mehrere Schuhinnenmaße (beispielsweise eine Länge und eine Breite, vorzugsweise eine Länge, eine Breite und eine Höhe, möglicherweise aber auch mehrere Längen, mehrere Breiten und/oder mehrere Höhen) und die entsprechenden Maße des Fußes vermessen. Das erfindungsgemäße Verfahren kann dadurch die zuverlässigere Bestimmung eines passenden Schuhs ermöglichen.

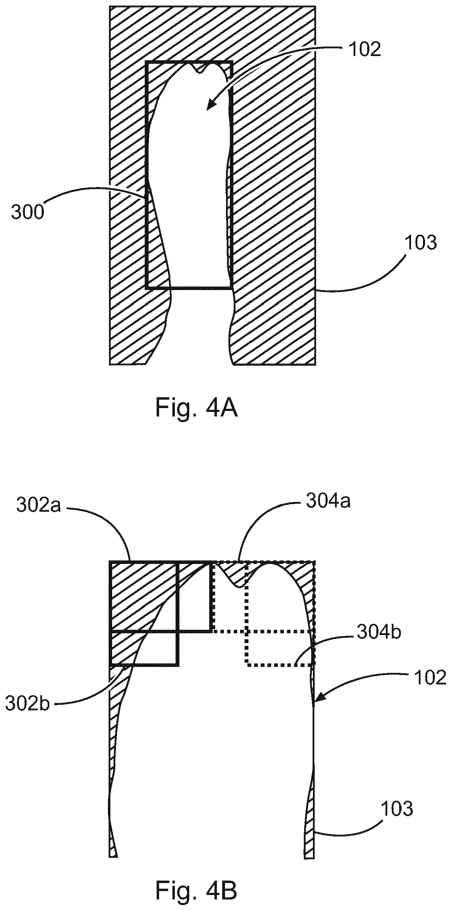

Figuren 4a bis 4d zeigen jeweils ein segmentiertes Bild zur Illustration von Schritten des Verfahrens zur Bestimmung eines anatomischen Maßes.

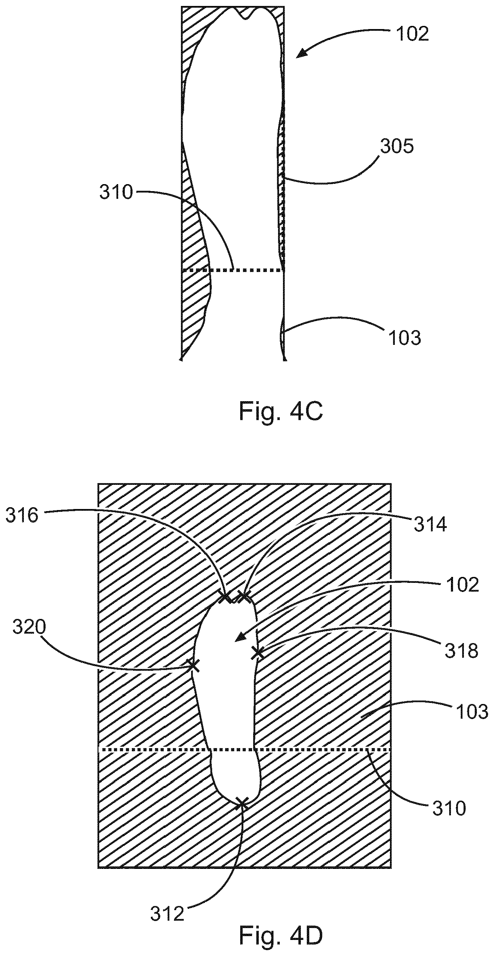

Figur 4a zeigt dabei eine Draufsicht auf den Fuß 102 eines Nutzers, beispielsweise wie in einem ersten Bild eines Videos festgehalten, wobei der Fuß 102 auf einem Blatt Papier 103 als Referenzobjekt steht. In dem Rahmen 300 in Figur 4a ist ein Ausschnitt eines von dem Server erkannten Vorderfußes bzw. einer Vorderfußzone gezeigt. Wie nachfolgend erläutert wird, kann ein Teil des segmentierten Bildes der Figur 4a mit einem oder mehreren weiteren segmentierten Bildern (oder Teilen davon) überlagert werden, um ein zusammengesetztes segmentiertes Bild zur Bestimmung des anatomischen Maßes zu generieren, wie in Figur 4d gezeigt.

Figur 4b zeigt einen Ausschnitt des Bildes aus Figur 4a, in welchem die Zehen des Fußes 102 erkennbar sind. Der Server kann dazu ausgeführt sein, eine Pixeldichte und/oder einen Pixelanteil von dem Fuß zugeordneten Pixeln des segmentierten Bildes in einem oder mehreren Bereichen 302a, 302b, 304a, 304b zu bestimmen. Beispielsweise können die Pixeldichten und/oder Pixelanteile in den Bereichen 302a, 304a und/oder 302b und 304b miteinander verglichen werden, um den Typ des Fußes, d.h. linker oder rechter Fuß, zu ermitteln. Ist die Pixelanzahl und/oder Pixeldichte in den Bereichen 304a, 304b höher als in den Bereichen 302a, 302b, so handelt es sich um den linken Fuß. Ist die Pixelanzahl und/oder Pixeldichte in den Bereichen 304a, 304b kleiner als in den Bereichen 302a, 302b, so handelt es sich um den rechten Fuß.

Zum Generieren des zusammengesetzten segmentierten Bildes der Figur 4d, welches zur Bestimmung des wenigstens einen anatomischen Maßes verwendet werden kann, kann der Server einen Anfang eines Beins des Nutzers bestimmen und/oder ermitteln. Hierzu kann der Server eine abrupte Vergrößerung der Breite des dem Fuß zugeordneten Bereichs des segmentierten (ersten oder weiteren) Bildes ermitteln, welche z.B. durch den Knöchel verursacht sein kann.

Der Server kann an einer Trennlinie 310 (siehe Figuren 4c und 4d), an der das Bein beginnt, senkrecht zur Innenfußtangente 305 (siehe Figur 4c) einen Teil des (ersten) segmentierten Bildes abschneiden und später durch ein, beispielsweise gleichermaßen mathematisch entzerrtes Bild des Fersenbereichs (z.B. so dass das Blatt Papier wieder vier 90°-Winkel an den Ecken hat) ergänzen. Referenz hierfür kann beispielsweise sein, dass die beiden zusammengefügten Teile in Summe in dem zusammengefügten segmentierten Bild die genormte DIN-A4-Blatt-Länge von 297mm ergeben.

Figur 4d zeigt ein kombiniertes bzw. zusammengesetztes segmentiertes Bild, in welchem ein Teil eines ersten segmentierten Bildes des Vorderfußes und ein Teil eines weiteren segmentierten Bildes des Fersenbereichs an der Schnittlinie 310 zusammengefügt und/oder überlagert sind. Im kombinierten bzw. zusammengesetzten segmentierten Bild kann optional zur Ermittlung des anatomischen Maßes, insbesondere der Fußlänge, der Hough-Circles-Algorithm und/oder eine Umfangsannäherung verwendet werden, um den Fersenpunkt 312, also den hintersten Teil des Fußes 102, in der Bildebene zu finden. Das Ergebnis dieses empirisch entwickelten Vorgehens kann dazu führen, dass der Fersenpunkt 312, beispielsweise wenn man ihn auf den 3D-Fuß zurück rechnet, in einer Höhe von ca. 0 bis 10 mm über der Aufstandsfläche bzw. dem Referenzobjekt 103 ermittelt wird. Diese Messhöhe korrespondiert mit der für die Schuhinnenlänge verwendeten Fußlängendefinition.

Der Server kann ferner dazu eingerichtet sein, den vordersten Punkt der Großzehe 314 und/oder den vordersten Punkt der zweiten Zehe (bzw. Zeigezehe) 316 zu ermitteln. Durch Bestimmung des Abstands der Punkte 314, 316 zum Fersenpunkt 312 in dem zusammengesetzten segmentierten Bild kann die Fußlänge als anatomisches Maß ermittelt werden. Alternativ oder zusätzlich kann die Fußlänge als Abstand eines Parallelenpaars bestimmt werden, das Fersenpunkt 312 und vordersten Punkt einer Zehe 314, 316 (Großzehe oder zweite Zehe je nach Fußform) berührt und ungefähr senkrecht zur Fußinnentangente 305 steht.

Alternativ oder zusätzlich kann der Server, insbesondere basierend auf der Fußinnentangente 305 und/oder einer Fußaußentangente, einen ersten Breitenmesspunkt 318 und einen zweiten Breitenmesspunkt 320 für die Bestimmung der Fußbreite ermitteln. Die Fußbreite kann dabei über den Abstand der beiden Breitenmesspunkte 318, 320 ermittelt werden.

Alternativ oder zusätzlich kann auch ein Umfangsmaß, etwa ein Fersenumfangsmaß und/oder ein Ballenumfangsmaß, bestimmt werden.

## Patentansprüche

1. Computer-implementiertes Verfahren (200) zur Bestimmung eines anatomischen Maßes, das Verfahren umfassend:
Laden eines ersten Bildes, wobei das erste Bild zumindest einen Körperteil (102) und ein Referenzobjekt (103) mit bekannten Abmessungen zeigt, und wobei eine Distanz des Referenzobjekts zu dem Körperteil in einer Bildaufnahmerichtung des ersten Bildes bekannt ist;
Bestimmen (214a) eines ersten segmentierten Bildes durch Anwenden eines konvolutionalen neuronalen Netzes auf das erste Bild, wobei das konvolutionale neuronale Netz dazu ausgeführt ist, den Körperteil, das Referenzobjekt sowie sonstige Bildabschnitte zu unterscheiden; und
Bestimmen des anatomischen Maßes auf der Grundlage des ersten segmentierten Bildes, der bekannten Abmessungen des Referenzobjekts und der bekannten Distanz des Referenzobjekts zu dem Körperteil in der Bildaufnahmerichtung.

2. Verfahren (200) nach Anspruch 1, wobei das Referenzobjekt (103) eine polygonale Grundfläche mit bekannten Abmessungen aufweist, und wobei das Verfahren ferner umfasst:
Bestimmen (215a) von ersten Eckpunkten der Grundfläche des Referenzobjekts in dem ersten segmentierten Bild;
Bestimmen (217a) eines ersten entzerrten Bildes durch Entzerren des ersten segmentierten Bildes unter Verwendung der ersten Eckpunkte; und
Bestimmen des anatomischen Maßes auf der Grundlage des ersten entzerrten Bildes, der bekannten Abmessungen des Referenzobjekts und der bekannten Distanz des Referenzobjekts zu dem Körperteil in der Bildaufnahmerichtung.

3. Verfahren (200) nach Anspruch 2, wobei die Grundfläche des Referenzobjekts (103) durch zwei Parallelenpaare begrenzt wird, und wobei das Bestimmen (215a) der ersten Eckpunkte der Grundfläche umfasst:
Bestimmen von vier Kanten der Grundfläche in dem ersten segmentierten Bild;
Zuordnen jeder der vier Kanten zu einem ersten oder einem zweiten Parallelenpaar; und
Bestimmen von ersten Schnittpunkten zwischen jeweils einer Kante des ersten Parallelenpaars und einer Kante des zweiten Parallelenpaars.

4. Verfahren nach einem der voranstehenden Ansprüche, ferner aufweisend:
Laden wenigstens eines weiteren Bildes, wobei das wenigstens eine weitere Bild den Körperteil und das Referenzobjekt in einer weiteren Bildaufnahmerichtung zeigen, welche sich von der Bildaufnahmerichtung des ersten Bildes unterscheidet;
Bestimmen wenigstens eines weiteren segmentierten Bildes durch Anwenden des konvolutionalen neuronalen Netzes auf das wenigstens eine weitere Bild; und
Überlagern wenigstens eines Teils des ersten segmentierten Bildes und wenigstens eines Teils des weiteren segmentierten Bildes unter Generieren eines zusammengesetzten segmentierten Bildes;
Wobei das anatomische Maß basierend auf dem zusammengesetzten segmentierten Bild bestimmt wird.

5. Verfahren nach Anspruch 4,
wobei das Überlagern ferner ein Bestimmen und/oder Ermitteln einer Gesamtfläche, einer Länge und/oder einer Breite des Referenzobjekts in dem zusammengesetzten segmentierten Bild und ein Vergleichen der bestimmten Gesamtfläche, der Länge und/oder der Breite des Referenzobjekts in dem zusammengesetzten segmentierten Bild mit einem Referenzmaß des Referenzobjekts.

6. Verfahren (200) nach einem der Ansprüche 2 bis 5, ferner umfassend:
Laden weiterer Bilder, wobei die weiteren Bilder den Körperteil und das Referenzobjekt mit der bekannten Distanz in der Bildaufnahmerichtung zeigen;
Bestimmen (214b, 214c, 214d) weiterer segmentierter Bilder durch separates Anwenden des konvolutionalen neuronalen Netzes auf die weiteren Bilder;
Bestimmen (215b, 215c, 215d) von weiteren Eckpunkten der Grundfläche des Referenzobjekts in den weiteren segmentierten Bildern;
Bestimmen (217b, 217c, 217d) weiterer entzerrter Bilder durch separates Entzerren von zumindest einem Teil der weiteren segmentierten Bilder unter Verwendung der weiteren Eckpunkte;
Bestimmen (218a, 218b, 218c, 218d) von Messwerten des anatomischen Maßes separat für das erste entzerrte Bild und die weiteren entzerrten Bilder auf der Grundlage der bekannten Abmessungen des Referenzobjekts und der bekannten Distanz des Referenzobjekts zu dem Körperteil in der Bildaufnahmerichtung; und
Bestimmen (219) des anatomischen Maßes auf der Grundlage der Messwerte.

7. Verfahren (200) nach Anspruch 6,
wobei das Bestimmen (219) des anatomischen Maßes auf der Grundlage der Messwerte das Bestimmen des Medians der Messwerte und/oder das Vergleichen der Messwerte umfasst.

8. Verfahren (200) nach Anspruch 6 oder 7, ferner umfassend:
Detektieren (216) von Ausreißern unter den ersten und/oder den weiteren Eckpunkten der Grundfläche in dem ersten und/oder den weiteren segmentierten Bildern.

9. Verfahren (200) nach Anspruch 8,
wobei eine Reihenfolge von Aufnahmezeitpunkten des ersten Bildes und der weiteren Bilder bekannt ist; und
wobei das Detektieren (216) von Ausreißern das Prädizieren und/oder Retrodizieren von Eckpunkten der Grundfläche auf der Grundlage der Reihenfolge der Aufnahmezeitpunkte des ersten Bildes und der weiteren Bilder umfasst.

10. Verfahren (200) nach einem der Ansprüche 6 bis 9, ferner umfassend
Bestimmen des ersten Bildes und der weiteren Bilder auf der Grundlage von nicht unmittelbar zeitlich aufeinander folgenden Einzelbildern (201 bis 212) eines Videos.

11. Verfahren (200) nach einem der vorangegangenen Ansprüche,
wobei das anatomische Maß eine Länge, eine Breite, ein Umfangsmaß, ein Fersenumfangsmaß, ein Ballenumfangsmaß oder eine Höhe eines menschlichen Fußes (102) ist.

12. Verfahren (200) nach einem der vorangegangenen Ansprüche, ferner umfassend:
Bestimmen (213a) des ersten Bildes durch Reduktion einer Auflösung eines von einer digitalen Kamera aufgenommenen Bildes (201).

13. Verfahren (200) nach einem der Ansprüche 2 bis 12, ferner umfassend:
Bestimmen einer Eignung einer Aufnahmerichtung des ersten Bildes relativ zu dem Körperteil für die Bestimmung des anatomischen Maßes auf der Grundlage der ersten Eckpunkte der Grundfläche des Referenzobjekts in dem ersten segmentierten Bild, den bekannten Abmessungen der Grundfläche sowie einer bekannten Anordnung des Körperteils relativ zu der Grundfläche.

14. Verfahren (300) zur Bestimmung eines Schuhmodells und einer Schuhgröße, das Verfahren umfassend:
Bestimmen (301) von Schuhinnenlängen, Schuhinnenbreiten und/oder Schuhinnenhöhen für eine Vielzahl von Schuhmodellen und Schuhgrößen;
Speichern (302) der Schuhinnenlängen, Schuhinnenbreiten und/oder Schuhinnenhöhen in einer Datenbank;
Bestimmen (303) einer Länge, einer Breite und/oder einer Höhe eines Fußes gemäß dem Verfahren nach einem der Ansprüche 1 bis 13; und
Durchsuchen (304) der Datenbank unter Berücksichtigung der Länge, der Breite und/oder der Höhe des Fußes, um das Schuhmodell und die Schuhgröße zu bestimmen.

15. Datenverarbeitungssystem umfassend Mittel zur Ausführung des Verfahrens (200) nach einem der Ansprüche 1 bis 14.
